# EUROPEAN PATENT SPECIFICATION

(11) **EP 3 027 056 B1**
(45) Date of publication and mention of the grant of the patent: **25.06.2025**
(21) Application number: 14733097.1
(22) Date of filing: 22.04.2014
(51) Int. Cl.: A23L 33/00, A23L 33/15, A23L 33/155, A61K 33/06, A61K 33/26, A61K 33/30, A61K 36/03, A61K 36/07, A61K 36/185, A61K 36/53

(54) **COMPOSITION FOR ENHANCING IMMUNITY**
ZUBEREITUNG ZUR VERBESSERUNG DER IMMUNITÄT
COMPOSITION POUR AMELIORER L'IMMUNITE

(30) Priority: 19.04.2013 EP 13164434; 17.12.2013 EP 13197725
(43) Date of publication of application: 08.06.2016
(73) Proprietor: IDC Investment Development Corporation Limited, Hong Kong (HK)
(72) Inventor: BOURGEOIS, Marie Françoise, 29530 Loqueffret (FR); CEFAÏ, Daniel, CH-1169 Yens (CH); WAHLI, Walter, CH-1112 Echichens (CH); PRINCE-DAVID, Mireille, BP 386 Lomé (TG); TUYISENGE, Lisine, BP 655 (RW)
(74) Representative: P&TS SA (AG, Ltd.)
(86) International application number: PCT/EP2014/058162
(87) International publication number: WO 2014/170505

(56) References cited:
- JP-A- 2009 221 112
- KR-A- 20110 121 088
- US-A1- 2004 146 545
- US-A1- 2006 078 629
- US-A1- 2008 038 367
- US-A1- 2012 034 201

## Description

### Field of the Invention

The invention relates to preparations for dietary, food supplement or medical purposes and more specifically to a safe and natural preparation or a composition comprising a combination of carefully selected natural supplements, which together are designed to be most effective in optimizing general health status and improving immunity and are useful for immunity regulation and/or stimulation. In particular the composition may provide for building, reinforcement, efficiency, maintenance and regeneration of natural immune defences in a subject and is also beneficial for improvement of sub-health conditions, without side effects.

### BACKGROUND OF THE INVENTION

### The immune system

The immune system is a complex and dynamic network of cells, membranes, organs, a circulatory system and many other components that all work together to recognize, attack and destroy microbes, bacteria, viruses, fungi and parasites that cause infections and diseases. The immune system also constantly scans our bodies for any sign of abnormal cell growth and intervenes to destroy those abnormalities.

There are many specialized white blood cells which work together to coordinate overall immune response. T cells are responsible for cellular immunity while B cells produce antibodies in response to antigens. The natural killer or NK cells can kill off certain body invaders such as bacteria and parasites by releasing toxic compounds. Macrophages can engulf and destroy foreign material. There are two major types of T cells, Th1 and Th2. Th1 cells drive immunogenic responses, while Th2 drive antibody production by B cells.

The success of the immune system relies on the timely and ordered function of all its components that constantly check their environment to trigger the adequate immunity.

### Nutrition and immunity

Nutrition, immunity and infection are inter-related (Bhaskaram P., Nutritional Review, 2002; 60(5): S40-5; Iliakis D and Kressig RW., The relationship between malnutrition and immunity, Ther. Umsch 2014 Jan;71(1):55-61). Malnourished persons show immune dysfunction, which predisposes them to infections. Conversely, proper micronutrient supplementation can enhance immune function and suppress infection.

Malnutrition and nutrient deficiencies are common in infectious diseases and are the leading underlying cause of immune deficiency (A. Niedzwiecki, M. Rath, Malnutrition: The Leading Cause of Immune Deficiency Diseases Worldwide, DRRI Brochure, 2005). Of the 13-14 million children dying annually in developing countries, most are malnourished and 70% die of infectious diseases. Immune deficiency as a consequence of malnutrition is the leading cause of death of children, elderly people and adults.

According to the World Health Organization, over 850 million people worldwide and 200 million adults in Sub-Saharan Africa suffer from malnutrition. Opportunistic infections and insufficient nutritional intake are part of a vicious cycle that contributes to immunodeficiency and impaired general health status that lead, in turn, to higher susceptibility to new infections. In a vicious circle, the resulting illness induces the loss of bodily nutrients, which further aggravate the pre-existing nutrient deficiencies. If no supplementation is provided, the vulnerability to pathogens increases, triggering a spiral of diseases very difficult to control. US 2004/146545 relates to a method of manufacturing micronutrition compositions comprising one or more active substances combined in a sequential manner in a pre-established order. US 2006/078629 relates to nutritional compositions comprising one or more selenium compounds in synergistic combination with substances enhancing physiological selenium absorption and utilisation for the treatment or prophylaxis of infections.

### Immunity reinforcement

Nutrients support numerous biochemical mechanisms underlying the function of the immune system: Non-specific defense components such as the synthesis of interferon and function of phagocytic cells (innate or natural immunity); Protective anti-microbial barriers created by the skin, mucus membranes (lungs, gut, ...), tears, saliva, gastric juice ...; Production of antibodies and optimization of cell-mediated immunity.

The components of the immune system rely on nutrients to mount and tune their responses to infectious agents. Well-nourished individuals are better prepared to fight infections.

Many infectious diseases can be prevented and their frequency and severity may be controlled by adequate nutrient supplementation with beneficial impact on the immune system.

### Nutraceuticals

Consumers are looking for ways to protect themselves from illness in the first place. Recent concerns address the possibility that dietary solutions readily available may protect them from illness in the first place. According to a study published in 2013 in the Journal of the American Medical Association, the most common reasons that adults reported using dietary supplements were to "improve overall health" (45%), to "maintain health" (33%), while boosting immunity and preventing colds was mentioned by 14.5% of adults. According to a 2012 study published in the Journal of the American Medical Association, immune health was one of the top three reasons consumers took food supplements.

### Food supplements: synergy towards optimal effect

It is widely accepted that maintaining a good nutritional status and adequate micronutrient stores in the body are essential for mounting an effective immune response to all kinds of infections. Micronutrients such as vitamin C, A, B-5, B-6, B-12, and folic acid as well as certain trace elements such as iron, zinc, selenium, copper, are essential for optimizing white blood cell production and immune function (Beisel WR, Am. J. Clin. Nutr.1982; 35: 415; Watson RR, Nutrition, disease resistance, and immunity. New York, Marcel Dekker, 1984).

It is also commonly accepted that a single nutrient does not ensure optimum health and a complete spectrum of micronutrients is needed to support the immune system to function at full capacity. Nutrient synergy is an approach that may result in the optimization of the overall cellular metabolism. Targeted nutrient synergy targeted to selected mechanisms may selectively restore and reinforce specific functions. Nutrient synergy is more effective than single nutrients, or their random combination, in addressing specific physiological tasks. Nutrient synergy can improve the synthesis and function of infection-fighting blood cells as well as stop infectious agents from spreading. Thus, nutrient synergy can provide effective health solutions through improving the general immunity of the body and supporting general health status. This can be achieved by compositions that include components that are individually active towards a desired goal but whose individual activities add up to a result that is remarkable, superior to the result that could be expected from the sum of their activities.

### Infectious diseases

According to the World Health Organization 2012 global report on infectious diseases, infectious diseases kill almost 9 million people every year, many of them children under five. They also cause enormous burdens through life-long disability. This represents one third of the mortality and 43% of deaths in developing countries.

Infectious diseases are caused by pathogenic microorganisms, such as bacteria, viruses, parasites or fungi. They can be spread, directly or indirectly, from one person to another.

Six main disease families are responsible for 99% of all deaths due to infectious diseases: Acute respiratory infections (including pneumonia and influenza) kill almost 3 million people per year, while Acquired ImmunoDeficiency Syndrome (AIDS) and diarrhoeal diseases each are responsible for 1.6 and 1 million deaths respectively. Tuberculosis affects mainly poor populations, causing up to 1.3 million deaths. Malaria and measles account each for about 1 million deaths per year.

Among these:
- Seasonal flu, according to the World Health Organization (WHO), is responsible for up to half a million deaths each year, representing nearly 13000 deaths each day. Three to five millions people are severely affected by this infection each year.
- The number of people suffering from Chronic obstructive bronchopneumopathy disease (COPD) is estimated at more than 64 million in the world. More than 3 million deaths were due to COPD in 2005 worldwide, representing 5% of all deaths this year.
- Diarrheal diseases account for 1 in 9 child deaths worldwide, making diarrhea the second cause of mortality in children below 5 worldwide. More than 1.7 billion cases of diarrhea are reported each year worldwide. Diarrhea kills 760000 children each year and is one of the leading causes of malnutrition in children below 5.
- Malaria is the leading parasitic infection in the world. According to the WHO, deaths due to malaria amount to more than 655000 each year while the Lancet journal indicated more than 1.2 million in 2010. The WHO reported more than 216 million malaria cases in 2010, of which 81% occurred in Africa, amounting to 174 million cases.
- Acquired Immune Deficiency Syndrome (AIDS) is a specific group of diseases or conditions that result from suppression of the immune system related to infection with the Human Immunodeficiency Virus (HIV). According to amfAR, the Foundation for AIDS Research, more than 35 million people now live with HIV/AIDS and 3.3 million of them are under the age of 15. In 2012, an estimated 2.3 million people were newly infected with HIV and 1.6 million people died from AIDS. Since the beginning of the epidemic, more than 75 million people have contracted HIV and nearly 36 million have died of HIV-related causes.

These infectious diseases result from the infection, presence and growth of pathogenic (capable of causing disease) biologic agents in an individual human or other animal host. Infections may range in severity from asymptomatic (without symptoms) to severe and fatal.

Despite a high number of specific treatments, therapies, prophylaxis and prevention, the high number of deaths due to infectious diseases indicates that such measures are inefficient or are not available - for a variety of reasons such as lack of supply, too high cost - to most individuals when they are most needed.

Fighting these infectious diseases may be achieved by prevention measures that aim at decreasing the occurrence and impact of such diseases on health of infected living beings. Prevention relies on 3 main pillars: avoiding infection, reinforcing immune defenses and prevention (prophylaxis measures) in case of risks of exposure to pathogens.

If it were possible to stimulate the immune system and improve general health status of all individuals, in particular those with decreased or impaired immune system or who exhibit higher susceptibility to infections or diseases, frequency, severity and duration of infections and infectious diseases could be diminished.

If it were possible to stimulate CD4 T cell recovery, normalize CD4/CD8 ratios in HIV infected patients and reverse the HIV-induced CD4 cell decline, improvement of clinical status could be accomplished.

The inventors believe that the use of readily available, cost-effective, natural and safe food supplements may act to stimulate the immune system of individuals, aid the ability of the body's immune system to fight infections and immune disorders, and would strengthen resistance of individuals to diseases and would be suitable to all.

Toward these objectives, the inventors have found that a nutraceutical composition comprising natural and safe ingredients described herein can be employed to effectively reinforce the immune system and prevent a wide spectrum of diseases and decrease their frequency, severity or duration.

### BRIEF DESCRIPTION OF THE INVENTION

One of the objects of the present invention is to provide a composition for the regulation and/or stimulation of the immune system of a subject for use in a method for the prevention and/or alleviation of the symptoms related to pathogenic infections comprising viral infections, bacterial infections, parasitic infections such as malaria, fungal infections, superinfections and episodic infections, comprising the combination of:
- 10µg to 1000µg (per 100g/100ml) of positively charged minerals consisting of magnesium,
- 10µg to 1000µg (per 100g/100ml) of metals selected from zinc and iron,
- 7µg to 700µg (per 100g/100ml) of at least one plant oil selected from *Ribes nigrum Oleum Acini* (blackcurrant seed oil) and *Elaeis guineensis* Oleum (palm oil),
- 6µg to 600µg (per 100g/100ml) of at least two plant extracts selected from *Thymus vulgaris* (thyme), *Cicer arietinum* (chickpea), and *Ervum lens* (lentil),
- 6µg to 600µg (per 100g/100ml) of at least one alga chosen among *Fucus vesiculosus* (Fucus), *Undaria pinnatifida* (Wakame), *Palmaria palmata* (Dulse), and *Porphyra umbilicalis* (Nori),
- 7µg to 700µg (per 100g/100ml) of at least one mushroom chosen among *Lentinula edodes* (Shiitake) and *Grifola frondosa* (Maitake),
- 8µg to 800µg (per 100g/100ml) of at least one vitamin chosen among vitamins A, B1, B2, B5, B6, B9, C, E, PP(B3);
optionally with a suitable excipient.

The present composition is particularly useful in the building, reinforcement, efficiency, maintenance and regeneration of natural immune defences of a subject. The present invention is active both in the regulation and/or stimulation of the innate immune system and the adaptive or acquired immune system.

The present invention is useful in the prevention and/or alleviation of the symptoms related to
- pathogenic infections comprising:
- parasitic infections such as malaria;
- episodic infections such as
winter infections, flu syndromes, bronchitis, tuberculosis as well as other lower and upper respiratory tract infections including Ear Nose and Throat (ENT) area, urinary, intestinal and skin infections.

The composition according to the invention is also useful in
the potentiating of antibiotics in pulmonary super infections such as pulmonary; ENT; urinary or skin infection,
the lowering in secondary infections due to chronic pathologies, and
the increase of mucosal immune defence comprising chronic obstructive bronchopneumopathy disease (COPD) and bronchiectasy.

Another object of the present invention is to provide a composition for the stimulation of natural defences and the immune system regeneration in a method for alleviating HIV infection and AIDS disorders.

A further object of the invention is to provide a composition useful in the regulation of inflammation due to a pathogen or an inappropriate immune response.

Another object of the invention is to provide a composition able to enhancing the immune response to antigenic proteins in vaccination or immunization protocols.

This invention concerns the implementation of a composition defined above usable as an additive or a food or nutritional supplement, and/or as a nutraceutical and/or therapeutic supplement, displaying properties which are particularly advantageous in the immunity regulation and/or stimulation. The composition of the invention is usable in the field of nutrition, in humans or in animals, and includes preventive care.

Other objects and advantages of the invention will become apparent to those skilled in the art from a review of the ensuing detailed description, which proceeds with reference to the following illustrative drawings, and the attendant claims.

### BRIEF DESCRIPTION OF THE FIGURES

Figure 1: CD4 cell count over 12 months according to treatment and initial viral charge.
Figure 2: Evolution of average increase in CD4 over 12 months resulting from treatment with the composition according to initial CD4 cell values.
Figure 3: Number of winter ENT-bronchitis infectious episodes per year, in the previous two years, illustrated as percentage of the population studied.
Figure 4: Reduction in the number of winter ENT-bronchitis infectious episodes during the year of immunity micronutrition intake, illustrated as percentage of the population studied.
Figure 5: Reduction in the number of malaria cases in children in the year of treatment with the composition of the invention.
Figure 6: Reduction in the number of flu cases in children in the year of treatment with the composition of the invention.
Figure 7: Reduction in the number of bronchitis cases in children in the year of treatment with the composition of the invention.
Figure 8: Reduction in the number of gut diseases cases in children in the year of treatment with the composition of the invention.
Figure 9A: Quantification of total cells, neutrophils, macrophages and lymphocytes in the bronchoalveolar fluid of mice infected with Influenza A virus at day 3 post-infection.
Figure 9B: Quantification of macrophages and lymphocytes in the bronchoalveolar fluid of mice infected with Influenza A virus at day 3 post-infection.
Figure 10: Quantification by quantitative polymerase chain reaction of the number of influenza virus RNA copies in lungs of mice infected with Influenza A virus at day 3 post-infection
Figure 11: Quantification of antibody titers to the P27A malaria antigenic protein in blood of mice immunized for 3 months with P27A.

### DETAILED DESCRIPTION OF THE INVENTION

Unless defined otherwise, all technical and scientific terms used herein have the same meaning as is commonly understood by one of skill in art to which the subject matter herein belongs. As used herein, the following definitions are supplied in order to facilitate the understanding of the present invention.

The term "comprise" is generally used in the sense of include, that is to say permitting the presence of one or more features or components.

As used in the specification and claims, the singular forms "a", "an" and "the" include plural references unless the context clearly dictates otherwise.

The term "extract", as used herein, includes any preparation obtained from plants, fruits or vegetables using an extraction method.

The term "food preparation" refers generally to material of either plant or animal origin, or of synthetic sources, that contain essential nutrients such as a carbohydrate, protein, fat, vitamin, mineral, etc. used in the body of an organism to sustain growth, repair, and vital processes and to furnish energy

A "dietary or food supplement" refers to a product that contains substances like vitamins, minerals, foods, botanicals, amino acids and is intended to supplement the usual intake of these substances. Dietary supplements are found in pill, tablet, capsule, powder or liquid form and are meant to be taken by mouth.

The term "nutraceutical" refers to any substance that is a food or a part of a food and provides medical or health benefits, including the prevention and treatment of disease. Such products may range from isolated nutrients, dietary supplements and specific diets to genetically engineered designer foods, herbal products, and processed foods such as cereals, soups and beverages. It also refers to a product isolated or purified from foods, and generally sold in medicinal forms not usually associated with food and demonstrated to have a physiological benefit or provide protection against diseases like chronic diseases for example.

The term "beverage" means a liquid for drinking, which may be water, flavoured water, soft drinks, alcoholic drink, health drink, or an enriched drink like based on a diary product (milk) or fruit juice.

"Pharmaceutically acceptable excipients or carriers" are any materials that do not interfere with the pharmacological activity of the active ingredient(s) or degrade the body functions of the subject to which it can be administered but facilitate fabrication of dosage forms or administration of the composition. Examples of pharmaceutically acceptable excipient include but are not limited to maltodextrin, calcium phosphate, and fused silica. Pharmaceutically acceptable excipients also include flavourings, as well as various additives such as other vitamins and minerals, all solvents, dispersion media, coatings, isotonic and absorption delaying agents, sweeteners and the like, non-toxic auxiliary substances such as wetting or emulsifying agents, pH buffering agents and the like, such as for example, sodium acetate, sorbitan monolaurate, triethanolamine oleate, and inert ingredients such as talc and magnesium stearate which are standard excipients in the manufacture of tablets, capsules and other dosage forms.

As used herein the terms "subject" or "patient" or "beneficiary" are well-recognized in the art, and, are used interchangeably herein to refer to a mammal, including dog, cat, rat, mouse, monkey, cow, horse, goat, sheep, pig, camel, and, most preferably, a human. In some embodiments, the subject is a subject in need of treatment or a subject with a disease or disorder. However, in other embodiments, the subject can be a normal subject. The term does not denote a particular age or sex. Thus, young, adult and new-born subjects, whether male or female, are intended to be covered.

The term "an effective amount" refers to an amount necessary to obtain a physiological effect. The physiological effect may be achieved by one application dose or by repeated applications. The dosage administered may, of course, vary depending upon known factors, such as the physiological characteristics of the particular composition; the age, health and weight of the subject; the nature and extent of the symptoms; the kind of concurrent treatment; the frequency of treatment; and the effect desired and can be effectively adjusted by a person skilled in the art.

The term "immunity regulation and/or stimulation" refers to the induction or enhancement of biological processes that result in timely and effective augmentation of immune defences against microbial infections or immune disorders and enables an infected subject or animal to:
- effectively fight an invading pathogen or immune disorders
- be prevented from being infected by such pathogen or suffering from such disorder, resulting in decreased susceptibility of the subject to such illness and in decreased frequency, severity or duration of such illness.
- gain, recover, or maintain good general health status that results in improved immunity

In another embodiment of the invention, the term "immunity regulation and/or stimulation" refers to enhancement of the immune response to an antigen after vaccination or immunization with this antigen.

In another embodiment of the invention, the term "immunity regulation and/or stimulation" refers to the regulation of inflammation, so as to prevent excessive immune response and tissue damage.

Applicants have developed innovative, efficacious and safe sequential micronutritional actives. These nutritional actives are micro-dosed. This so-called combinatorial micronutrition is based on a sequential assembly of low amounts of precise ingredients (trace elements, different oils, vitamins) which consist of food products made in compliance with the Swiss, French and European regulations.

The results as presented in the Examples show a significant ability of the composition of the invention to reinforce the immune status of beneficiaries. This strengthening is pivotal in the prevention of diseases, in the accompaniment of therapies as well as in the reinforcement of weakened natural immune defences.

Applicants have surprisingly discovered that the safe and natural composition according to the invention shows an interesting potential in immunity regulation and/or stimulation.

The "immune system" is the collection of cells, tissues and molecules that protects the body from numerous pathogenic microbes and toxins in our environment. This defence against microbes has been divided into two general types of reactions: reactions of innate immunity and reactions of adaptive immunity also known as acquired immunity.
- The innate immune system constitutes the first line of defence against harmful foreign substances and pathogenic microorganisms. It is an immediate, nonspecific defense that consists of cells and proteins that are always present and ready to mobilize and fight microbes at the site of infection. The main components of the innate immune system are physical epithelial barriers, phagocytic leukocytes, dendritic cells, a special type of lymphocyte called a natural killer (NK) cell, and circulating plasma proteins.
- The adaptive immune system involves antigen-specific responses and immunologic memory. It is called into action against pathogens that are able to evade or overcome innate immune defences. It deals with specific fragments of any potentially harmful or foreign material. These fragments, called antigens, are specifically recognized by specialized structures on T cells that are called antigen receptors. Components of the adaptive immune system are normally silent. However, when it is activated, an antigen receptor adapts to one of these invading fragments and triggers specific defence mechanisms. Moreover, it retains antigenic memory so that it may quickly neutralize that antigen if it invades the body again. There are two types of adaptive immune responses: humoral immunity, mediated by antibodies produced by B lymphocytes, and cell-mediated immunity, mediated by T lymphocytes.

Those skilled in the art will appreciate that the scope of the composition of the invention, as it acts to reinforce the natural immune defences of a subject, is not to be considered restricted to some infectious diseases but rather include all infectious diseases caused by pathogens such as bacteria, both gram-negative or gram-positive bacteria, viruses, fungi, protozoa, and parasites. Some of these disease agents can be transmitted from animals to humans or from humans to humans, and some of these agents can be transmitted in more than one way. Such agents may affect the lungs, the Ear Nose and Throat (ENT) area, the gut and intestinal tract, the liver, the kidneys, the skin, the urinary system, the bloodstream, the lymphoid organs and the mucosal surfaces of a subject and reach the bloodstream of said subject, causing subsequent infectious diseases in the general population, and in particular in weakened or immunocompromised subjects.

In one aspect of the present invention there is provided a composition for immunity regulation and/or stimulation including the building, reinforcement, efficiency, maintenance and regeneration of natural immune defences, for use in a method for the prevention and/or alleviation of the symptoms related to pathogenic infections comprising viral infections, bacterial infections, parasitic infections such as malaria, fungal infections, superinfections and episodic infections, said composition comprising the combination of:
- 10µg to 1000µg (per 100g/100ml) of positively charged minerals consisting of magnesium,
- 10µg to 1000µg (per 100g/100ml) of metals selected from zinc and iron,
- 7µg to 700µg (per 100g/100ml) of at least one plant oil selected from *Ribes nigrum Oleum Acini* (blackcurrant seed oil) and *Elaeis guineensis* Oleum (palm oil),
- 6µg to 600µg (per 100g/100ml) of at least two plant extracts selected from *Thymus vulgaris* (thyme), *Cicer arietinum* (chickpea), and *Ervum lens* (lentil),
- 6µg to 600µg (per 100g/100ml) of at least one alga chosen among *Fucus vesiculosus* (Fucus), *Undaria pinnatifida* (Wakame), *Palmaria palmata* (Dulse), and *Porphyra umbilicalis* (Nori),
- 7µg to 700µg (per 100g/100ml) of at least one mushroom chosen among *Lentinula edodes* (Shiitake) and *Grifola frondosa* (Maitake),
- 8µg to 800µg (per 100g/100ml) of at least one vitamin chosen among vitamins A, B1, B2, B5, B6, B9, C, E, PP(B3);
optionally with a suitable excipient.

Preferably, the excipient is a pharmaceutical and/or alimentary (nutritionally) acceptable excipient. Said excipient will advantageously supplement the volume so as to obtain 100 ml or 100g of the composition in accordance with the invention.

More preferably the above composition for immunity regulation and/or stimulation, per 100 g or 100 ml, consists in the combination of:
- 10µg to 1000µg of magnesium,
- 10µg to 1000µg of metals selected from zinc and iron,
- 7µg to 700µg of at least one plant oil selected from *Ribes nigrum Oleum Acini* and *Elaeis guineensis Oleum,*
- 6µg to 600µg of at least two plant extracts selected from *Thymus vulgaris, Cicer arietinum,* and *Ervum lens,*
- 6µg to 600µg of at least one alga selected from the group consisting *of Fucus vesiculosus, Undaria pinnatifida, Palmaria palmate* and *Porphyra umbilicalis,*
- 7µg to 700µg of at least one mycelium or mycelium extracts selected from the group consisting of *Lentinula edodes* and *Grifola frondosa,*
   - 8µg to 800µg of at least one vitamin selected from the group consisting of vitamins A, B1, B2, B5, B6, B9, C, E, PP(B3);
and a pharmaceutically and/or nutritionally acceptable excipient.

Preferably the composition for immunity regulation or prevention contains vitamin A comprised between 15% to 100% of the GDA (Guideline Daily Amounts). In particular the weight of vitamin A represents from 120 to 800 µg/100g of said composition and preferably from 120 to 800 µg/100g of said composition. 800 µg/day of vitamin A being the GDA (Guideline Daily Amount).

In a preferred embodiment, the composition for immunity regulation and/or stimulation comprises zinc between 15% to 100% of the GDA (Guideline Daily Amounts). In particular, the weight of zinc represents from 0.225 to 1 mg/100g of said composition.

In another aspect, the present invention provides for a dietary or food supplement, a food preparation, a beverage, and a medicament comprising the preparation of the present invention.

The object of the present invention is to provide the composition for immunity regulation and/or stimulation in the prevention and/or alleviation of the symptoms related to pathogenic infections comprising parasitic infections such as malaria and episodic infections.

Parasites are microorganisms that live off of other organisms, or hosts, to survive. Some parasites don't affect the host, while others grow, reproduce, or give off toxins that make the host sick resulting in a parasitic infection.

Parasitic infections represent a large health problem. They are common in rural or developing areas of Africa, Asia, and Latin America, in tropical and subtropical regions, and less common in developed areas. The composition of the present invention is particularly effective on the treatment, the prevention and/or alleviation of the symptoms due to parasitic infections such as malaria or other parasitic including trichomoniasis, giardiasis, cryptosporidiosis, and toxoplamosis.

Parasites that infect people include protozoa (such as amebas), which consist of only one cell, and worms (helminths, such as hookworms and tapeworms), which are larger and consist of many cells and have internal organs. Protozoa, which reproduce by cell division, can reproduce inside people. Helminths, in contrast, produce eggs or larvae that develop in the environment before they become capable of infecting people.

Malaria is one of the deadliest diseases caused by a parasite. Plasmodium falciparum, the causative agent of malaria, is a major public health problem principally in sub-Saharan African countries. Most common plasmodium species include Plasmodium falciparum, the deadliest species, Plasmodium ovale, Plasmodium vivax and Plasmodium malariae. Plasmodium is transmitted to humans following a sting by the Anopheles mosquito which serves as vector for the parasite. The falciparum parasites pass through several stages of development: the infectious form of the parasites injected by the mosquito, sporozoites, enter the body, reach the bloodstream and invade first the liver, where they replicate intensively to yield merozoites that in turn invade erythrocytes and multiply, to yield gametocytes (sexual stages) that may be taken up by a mosquito sting, completing the parasite life cycle. All these stages have their own unique shapes and structures and protein complements. The surface proteins and metabolic pathways keep changing during these different stages that help the parasite to evade the immune clearance, while also creating problems for the development of drugs and vaccines.

Symptoms of malaria include fever (above 38°C), shivering, headaches, fatigue, sweats, muscle ache, nausea, diarrhea and vomiting. Severe malaria, almost exclusively caused by Plasmodium falciparum, can lead to anemia, brain damage and death.

The development of a safe and effective malaria vaccine remains an urgent unmet medical need. An effective vaccine is expected to be one that not only reduces the risk of infection by Plasmodium falciparum sporozoites, but also induces immunity against Plasmodium falciparum blood stage parasites and protects against morbidity and mortality from the disease, especially by triggering the production of specific antibodies. According to WHO, the targeted protection level of 75% of the population using vaccine and treatment strategies will not be reached by 2030.

Recurrent or "episodic infections" are infections that are too great in number, too severe, or too long lasting. Recurrent infections are defined as two or more severe infections in one year, three or more respiratory infections (eg, sinusitis, otitis, bronchitis) in one year, or the need for antibiotics for two months/year. Severe/serious infections include those with persistent fever or confinement to bed for a week or more, failure to respond to oral antibiotics and/or the need for intravenous antibiotics or hospitalization, infections with an unusual pathogen, or unusual complications (eg mastoiditis, pleural effusion, abscesses).

Preferably the episodic infections comprise the winter infections, flu syndromes, bronchitis, tuberculosis, lower and upper respiratory tract infections including ENT, the urinary, intestinal, mucosal and skin infections.

Influenza (Flu), caused by influenza virus types A, B, and C, is a respiratory disease that may have dramatic consequences, especially in immunocompromised or weakened subjects. The flu is a highly contagious disease: the flu virus is spread through inhalation of infected droplets in the air (spread when an infected person coughs or sneezes) or direct contact with an infected person's secretions (by kissing, touching, sharing objects such as spoons and forks). Flu outbreaks are classified as epidemics, which means they occur in a set geographical area, or pandemics, which means a worldwide occurrence. According to the CDC, winter flu epidemics can cause illness in 10% to 20% of the population and are associated with 3,000 to 49,000 deaths and more than 200,000 hospitalizations per year in the United States.

Flu prevention includes washing hands, avoiding spreading droplets through sneezing and also by a vaccination. Protection is mediated by the induction of antibody production. Vaccination is particularly advised for high-risk individuals who are more prone to flu complications, such as pneumonia. However, conferred protection usually does not reach 100% and vaccinated people can catch the flu, although with milder infectious symptoms.

Bronchitis is an inflammation of the lining of the bronchial tubes. It may be either acute or chronic. Acute bronchitis often develops from a cold or other respiratory infection, and is very common. Chronic bronchitis, a more serious condition, is a constant irritation or inflammation of the lining of the bronchial tubes, often due to smoking. Acute bronchitis usually improves within a few days without lasting effects. However, repeated bouts of bronchitis suggest a chronic bronchitis, which requires medical attention. Chronic bronchitis is one of the conditions included in chronic obstructive pulmonary disease (COPD). Treatment for bronchitis focuses on relieving symptoms and easing breathing.

Tuberculosis (TB) is an infection caused by the Mycobacterium Tuberculosis bacterium. TB usually affects the lungs but any part of the body can be affected. Anyone can develop active TB, but you are more likely to develop it if you are already in poor health or have a poor immune system. With treatment, most cases are cured. Without treatment, TB can be fatal. TB bacteria are coughed or sneezed into the air by people with active TB disease. Contagion occurs through breathing of tiny water droplets containing the bacterium. Infection can progress in three ways: Minor infection with no symptoms occurs in most cases as bacteria are eliminated by the immune system of the host; Infection may progress into active TB disease in about 1 in 20 people who breathe in some TB bacteria, mainly due to a weak immune response, as is observed in malnourished children in developing countries; Reactivated (secondary) infection due to the fact that not all TB bacteria have been killed in a previous infection can cause active disease. Dormant TB bacteria may start to multiply and cause active TB if the body's immune system begins to fail later in life. This occurs preferentially in elderly, frail, diabetic, AIDS or in malnourished subjects.

Lower Respiratory tract Infections mainly consist of bronchitis, bronchiolitis and pneumonia. The causative agents are viral or bacterial. Viruses cause most cases of bronchitis and bronchiolitis. In community-acquired pneumonias, the most common bacterial agent is *Streptococcus pneumoniae.* Atypical pneumonias are cause by such agents as *Mycoplasma pneumoniae, Chlamydia spp, Legionella, Coxiella burnetti* and viruses. Infectious viruses or bacteria enter the distal airway by inhalation, aspiration or by hematogenous seeding. The pathogen multiplies in or on the epithelium, causing inflammation, increased mucus secretion, and impaired mucociliary function; other lung functions may also be affected. In severe bronchiolitis, inflammation and necrosis of the epithelium may block small airways leading to airway obstruction.

Symptoms include cough, fever, chest pain, tachypnea and sputum production. Patients with pneumonia may also exhibit non-respiratory symptoms such as confusion, headache, myalgia, abdominal pain, nausea, vomiting and diarrhea.

Symptomatic treatment is used for most viral infections. Bacterial pneumonias are treated with antibacterials. A polysaccharide vaccine against 23 serotypes of *Streptococcus pneumoniae* is recommended for individuals at high risk.

Upper respiratory tract infections are illnesses caused by an acute infection which involves the upper respiratory tract: nose, sinuses, pharynx or larynx. This commonly includes: Rhinitis, tonsillitis, (naso)pharyngitis, laryngitis, (rhino)sinusitis, otitis media, epiglottitis, laryngotracheitis, tracheitis and the common cold. They are associated with symptoms such as cough, sore throat, runny nose, nasal congestion, headache, low grade fever, facial pressure and sneezing.

Most upper respiratory infections are of viral etiology. Over 200 different viruses have been isolated in patients with Upper respiratory tract infections. The most common viruses are the rhinovirus family. Other viruses include the coronavirus, parainfluenza virus, adenovirus, enterovirus, and respiratory syncytial virus.

Epiglottitis and laryngotracheitis are exceptions to the viral etiology, with severe cases likely caused by Haemophilus influenzae type b. Up to 15% of acute pharyngitis cases may be caused by bacteria, most commonly Streptococcus pyogenes, a Group A streptococcus in Streptococcal pharyngitis. Other bacterial causes are streptococcus pneumoniae, haemophilus influenzae, corynebacterium diphtheriae, bordetella pertussis, and bacillus anthracis.

Infectious viruses or bacteria gain entry to the respiratory tract by inhalation of droplets and invade the mucosa. Epithelial destruction may ensue, along with redness, oedema, haemorrhage and sometimes an exudate.

Intestinal infections affect the gastrointestinal tract, often causing diarrhea.

Gastroenteritis, an inflammation of the stomach and intestines, frequently accompanies such infections. Bacteria, viruses and parasites can cause intestinal infections.

Infectious bacteria include:
- The Salmonella family. Salmonella typhi causes typhoid, with severe symptoms such as high fever, headache, extreme tiredness or weakness, stomach pain, loss of appetite, and sometimes a flat, red rash. Salmonella can also cause salmonellosis with symptoms that include nausea, vomiting, diarrhea, fever, and stomach cramps. Shigella
- The Shigella family that causes shigellosis and inflames the lining of the small intestine. Symptoms of shigellosis include diarrhea (sometimes with blood or mucus), fever, vomiting, nausea, and abdominal cramping.
- Escherichia coli: out of the hundreds of types of Escherichia coli bacteria, only five are known to cause illness in people. In the United States, the Center for Disease Control (CDC) estimates that there are 73,000 cases of E. coli infection, leading to about 60 deaths, each year. E. coli infection can cause abdominal cramps and bloody diarrhea, which last about 5 days. Most people do not need treatment, although those with weak immune systems, children, and the elderly will need to be hospitalized if they develop a serious infection.
- Campylobacter cause campylobacteriosis, the most common type of bacterial diarrhea in the United States. Campylobacter jejuni causes about 99 percent of these cases. The CDC estimates that more than 2 million people, or almost 1 percent of the U.S. population, contract the infection each year.
- *Clostridium difficile* bacteria often live in the intestinal tracts of infants and young children without causing disease. In adults, however, especially the elderly, *C*. *difficile* can produce fever, watery diarrhea, abdominal pain, and loss of appetite.
- Perfringens poisoning is caused by the *Clostridium perfringens* bacterium and is a common types of food poisoning. Some *C. perfringens* bacteria may remain in food even after it has been cooked, then multiply when the food is cooled slowly and left at room temperature.
- Listeriosis is caused by the Listeria monocytogenes bacterium, which is found in the soil and in the intestinal tracts of animals and humans. Symptoms of illness include fever, headache, nausea, and diarrhea. The bacteria also can spread into the bloodstream or nervous system, leading to meningitis.
- Staphylococcus: Toxins produced by certain strains of *Staphylococcus aureus* bacteria can cause food poisoning. Symptoms include severe nausea and vomiting and sometimes diarrhea.

Main viral causes of intestinal infections include:
- Rotaviruses can infect people of all ages, but infants and young children are infected most often. They affect about 1 million children each year in the United States. Of those, between 55,000 and 70,000 require hospitalization. Worldwide there are more than 600,000 deaths among children each year from rotavirus infection, according to WHO. Rotaviruses spread when people come into contact with infected human feces. Symptoms include fever, vomiting, and abdominal pain, and diarrhea, which can linger for up to 8 days.
- Enteroviruses are a group of viruses that attack the intestinal tract and cause a wide range of illnesses, including intestinal infections. People who are infected may experience mild diarrhea, vomiting, fever, and abdominal pain.
- The hepatitis A virus is found in water contaminated by sewage, in shellfish from contaminated water, and in fruits and vegetables grown in contaminated soil. The virus can spread when people eat or drink contaminated food or water or from person to person during sexual intercourse. Some people with hepatitis A infection show no signs of illness, but those who do may experience fever, extreme tiredness, loss of appetite, nausea, and vomiting. The patient's liver enlarges and the skin may appear yellowish, a condition known as jaundice. The disease can lead to permanent liver damage, although this is rare. Symptoms appear 2 to 4 weeks after infection and may last several weeks to months.

Parasitic intestinal infections include amebiasis, caused by Entamoeba histolytica; giardiasis, caused by the Giardia intestinalis protozoa; and infection with *Cyclospora cayetanensis.* These parasitic infections lead to intestinal symptoms such as cramping and diarrhea. Intestinal infections are very common, particularly in developing parts of the world. The World Health Organization (WHO) estimates that about 2 million children worldwide die each year from diseases that cause diarrhea. Children, the elderly, and people who have weak immune systems are most likely to contract intestinal infections.

Urinary tract infections (UTIs) occur when one or more parts of the urinary system (kidneys, ureters, bladder, or urethra) become infected with a pathogen (most frequently, bacteria). UTIs most commonly occur in females; about 50% of all females get a urinary tract infection during their lifetime. Bladder infections are the most common type. If the UTI reaches the kidneys, serious illness, and even death, can occur, especially if pathogens enter the bloodstream.

An object of the present invention is to provide the composition for immunity regulation and/or stimulation in the prevention and/or alleviation of the symptoms related to eye infections.

Eye infections are typically caused by bacteria, fungi, or viruses. Eye infections can occur in different parts of the eye and can affect just one eye or both. Symptoms of eye infections may include redness, itching, swelling, discharge, pain, or problems with vision. Treatment depends on the cause of the infection and may include eye drops, creams, or antibiotics. Two common eye infections are conjunctivitis and stye.
- Stye is a bump on the eyelid occurring when bacteria from the skin get into the hair follicle of an eyelash.
- Conjunctivitis, also known as pinkeye, is often due to an infection. It is very contagious. Conjunctivitis can result from many causes. These causes include viruses, bacteria, allergens, contact lens use (especially the extended-wear type), chemicals, fungi, and certain diseases. Viral Conjunctivitis can be caused by viruses such as Adenoviruses, Rubella virus, Picornaviruses, such as enterovirus 70 and coxsackievirus A24, Rubeola (measles) virus, Herpes viruses, including herpes simplex virus, varicella-zoster virus, or Epstein-Barr virus. Viral conjunctivitis is highly contagious. Most viruses that cause conjunctivitis are spread through hand-to-eye contact by hands or objects that are contaminated with the infectious virus. Hands can become contaminated by coming in contact with infectious tears, eye discharge, fecal matter, or respiratory discharges.
- Bacterial Conjunctivitis can be caused by *Staphylococcus aureus, Haemophilus influenzae Streptococcus pneumoniae,* or Moraxella catarrhalis.

Bacterial conjunctivitis is highly contagious. Most bacteria that cause conjunctivitis are spread through direct hand-to-eye contact from contaminated hands.

Globally, the bacterium Chlamydia trachomatis (trachoma) is the leading cause of preventable blindness of infectious origin. Trachoma is a chronic follicular conjunctivitis, which is transmitted from person-to-person, through shared items or by flies. Topical antimicrobial therapy is indicated for bacterial conjunctivitis, usually distinguished by a purulent exudate. Infectious conjunctivitis (viral or bacterial) can also be spread by large respiratory tract droplets. Bacterial conjunctivitis is less common in children older than 5 years of age.
- Allergic conjunctivitis is common in people who have other signs of allergic disease, such as hay fever, asthma, and eczema. It is caused by the body's reaction to certain substances to which it is allergic, such as pollen from trees, plants, grasses, and weeds, dust mites, animal dander, molds, contact lenses and lens solution or cosmetics.

Skin infections comprise bacterial, fungal, and viral skin infections.
- Bacterial skin infections are mainly three types:
   Impetigo, highly contagious, is primarily caused by *Staphylococcus aureus,* and sometimes by *Streptococcus pyogenes.* It is the major skin infection in children.
   Erysipelas is an acute streptococcus bacterial infection of the deep epidermis; and
   Cellulitis is a diffuse inflammation of connective tissue with severe inflammation of dermal and subcutaneous layers of the skin.
   Leprosy is an important skin infection, caused by *Mycobacterium leprae.*
- Viral skin infections include Molluscum Contagiosum, Shingles (herpes zoster) and Chickenpox (varicella), characterized by a very itchy red rash and one of the most common infectious diseases of childhood.
- Fungal Skin Infections include athlete's foot, jock itch, ringworm (caused by dermatophytes), yeast infections (candidiasis, Sporotrichosis) and mycoses.

A mycosis is a common fungal infection of animals, including humans. Inhalation of fungal spores or localized colonization of the skin may initiate persistent infections; therefore, mycoses often start in the lungs or on the skin. Individuals with weakened immune systems are at risk of developing fungal infections. This is the case of people with HIV/AIDS, under steroid treatments, taking chemotherapy, with diabetes and very young and very old people.

An object of the present invention is to provide the composition for immunity regulation and/or stimulation in the prevention and/or alleviation of the symptoms related to Herpes simplex infections.

Herpes simplex is a viral disease from the herpesviridae family caused by both Herpes simplex virus type 1 (HSV-1) and type 2 (HSV-2). Infection with the herpes virus is categorized into one of several distinct disorders based on the site of infection. Oral herpes, the visible symptoms of which are colloquially called cold sores or fever blisters, is an infection of the face or mouth. Oral herpes is the most common form of infection. Genital herpes, known simply as herpes, is the second most common form of herpes. Other disorders such as herpetic whitlow, herpes gladiatorum, ocular herpes, cerebral herpes infection encephalitis, Mollaret's meningitis, neonatal herpes are all caused by herpes simplex viruses. Herpes simplex is most easily transmitted by direct contact with a lesion or the body fluid of an infected individual. Transmission may also occur through skin-to-skin contact during periods of asymptomatic shedding.

Barrier protection methods are the most reliable method of preventing transmission of herpes, but they merely reduce rather than eliminate risk.

A cure for herpes has not yet been developed. Once infected, the virus remains in the body for life. Recurrent infections (outbreaks) may occur from time to time, especially in times of immune impairment such as HIV and cancer-related immune suppression. Treatments with antivirals can reduce viral shedding and alleviate the severity of symptomatic episodes.

Another object of the invention is to use said composition for immunity regulation and/or stimulation for the potentiating the action of antibiotics in pulmonary superinfections comprising pulmonary; Ear Nose and Throat (ENT); urinary tract or skin infections.

"Superinfections" refer to a new infection occurring in a patient having a pre-existing infection, such as bacterial super-infection in viral respiratory disease.

Virus infections of the respiratory tract predispose it to bacterial superinfections. It appears that structural and functional disruption of the respiratory mucosal epithelium is a major contributor to the synergistic effects of super-infection: the enhanced susceptibility to bacterial infections following viral lung colonization suggests a general impairment of pulmonary antibacterial defences brought about by the viral infection.

Current research suggests that the flu may predispose to secondary bacterial infections, which account for a significant proportion of mortality during flu pandemics. The superinfection by bacteria greatly increases the morbidity and mortality of the disease. The most common bacterial agents found following flu pandemics have been *Streptococcus pneumoniae, Haemophilus influenzae,* Group A Streptococcus, and *Staphylococcus aureus.*

At highest risk for a superinfection are people with respiratory problems, such as asthma, chronic obstructive pulmonary disease, emphysema and smoking habits, and those with compromised immune systems, like diabetics, pregnant women, dialysis patients and even obese individuals.

A further object of the invention is to use said composition for immunity regulation and/or stimulation in a method for the lowering in secondary infections due to chronic pathologies.

Chronic Disease or pathology is a long-lasting condition that can be controlled but not cured. Chronic illness affects the population worldwide, making subjects more susceptible to opportunistic or recurrent infections.

Chronic Obstructive Pulmonary Disease (COPD) is a chronic lung disease that makes it hard to breathe. It is caused by damage to the lungs over many years, usually from smoking. COPD is often a mix of two diseases, namely chronic bronchitis, with inflammation of the bronchial tubes and mucus secretion, and emphysema, with damaged airways and shortness of breath. COPD gets worse over time. The damage to the lungs is irreversible.

COPD is almost always caused by smoking. Over time, breathing tobacco smoke irritates the airways and destroys the stretchy fibers in the lungs.

The main symptoms are a long-lasting (chronic) cough and mucus secretion and shortness of breath. COPD exacerbation can range from mild to life-threatening.

In COPD, compromised mucociliary and barrier functions may increase virus binding and allow virus entry into airway epithelial cells, making exacerbations associated with respiratory viruses more severe in COPD subjects.

The composition of the present invention is also useful in a method for increasing the mucosal immune defence. In particular the composition of the invention may be used in the treatment or prevention of respiratory infections, in particular chronic obstructive bronchopneumopathy disease (COPD) or Bronchiectasy, and urinary or intestinal infections.

The "mucosal immune system" is that portion of the immune system which provides protection to an organism's various mucous membranes from invasion by potentially pathogenic microbes. It provides three main functions protecting the mucus membrane against infection, preventing the uptake of antigens, microorganisms, and other foreign materials, and moderating the organism's immune response to that material.

The present invention describes a composition for the protection from infection and/or the treatment before infection by an inhaled pathogen. Said pathogen can be a viral, bacterial, fungal, etc. that comes in contact with a subject or an animal. In certain aspects the invention, the composition does not require an adaptive or acquired immune response to be effective, and therefore can be used in immunocompromised subjects and against a broad spectrum of pathogenic or potentially pathogenic organisms.

Besides, the composition of the invention is provided in the alleviation of HIV infection and AIDS disorders.

A person infected with HIV gradually loses immune function along with immune cells called CD4 T lymphocytes or CD4 T cells that play a central role in the immune response, causing the infected person to become vulnerable to opportunistic infections such as pneumonia, fungus infections, and other common ailments.

Another embodiment of the composition for immunity regulation and/or stimulation according to the invention comprises its use in a method for the regulation of inflammation due to an inappropriate immune response or an infection.

"Inflammation" is part of the complex biological response of vascular tissues to harmful stimuli, such as pathogens, damaged cells, or irritants. The inflammatory response enables a subject to immediately detect and destroy infection or toxic material in damaged tissue before it can spread to other areas of the body. Inflammation develops as a normal protective response of the immune system when body tissue is irritated. When tissue is irritated, the immune system increases blood flow to the area, which causes localized swelling, warmth, and redness. Inflammation may occur anywhere in the body, and may occur with overuse of a body area or with minor injuries.

Inflammation is a protective attempt by the organism to remove the injurious stimuli and to initiate the healing process. Inflammation is not a synonym for infection, even in cases where inflammation is caused by infection.

Although infection is caused by a microorganism, inflammation is one of the responses of the organism to the pathogen. However, inflammation can be considered as a mechanism of innate immunity as compared to adaptive immunity, which is specific for each pathogen.

Inflammation can be classified as either acute or chronic:
- Acute inflammation is the initial response of the body to harmful stimuli and is achieved by the increased movement of plasma and leukocytes (especially granulocytes) from the blood into the injured tissues. A cascade of biochemical events propagates and matures the inflammatory response, involving the local vascular system, the immune system, and various cells within the injured tissue. The classical signs of acute inflammation are pain, heat, redness, swelling, and loss of function.

Acute inflammation may also occur as a result of acute infection. The inflammatory response to influenza virus infection in vivo is a major component of the damaging immunopathology associated with fatal infections (JK Taubenberger and DM Morens. The Pathology of Influenza Virus Infections. Annual Review of Pathology. 2008, Vol. 3: 499-522).
- Prolonged inflammation, known as chronic inflammation, leads to a progressive shift in the type of cells present at the site of inflammation and is characterized by simultaneous destruction and healing of the tissue from the inflammatory process. Progressive destruction of the tissue would compromise the survival of the organism. It is becoming increasingly clear that chronic inflammation can lead to a host of diseases, such as hay fever, atherosclerosis, rheumatoid arthritis, heart disease, Alzheimer's disease and even cancer (e.g., gallbladder carcinoma). For that reason, inflammation is normally closely regulated by the body.

Chronic inflammation stems from an imbalance between the innate and acquired immune systems. Pro-inflammatory cells proliferate and can damage healthy cells -- in the blood vessels (atherosclerosis) -- the pancreas (diabetes) -- the joints (arthritis), and others areas - the auto-immune response. Symptoms of excess inflammation include body aches and pains, congestion, frequent infections, diarrhea, dry eyes, indigestion, shortness of breath, skin outbreaks, swelling, stiffness or weight gain/obesity. Chronic low-grade inflammation is thought to be one of the leading causes of disease, premature aging and illness.

Immune system disorders occur when the immune response is inappropriate, excessive, or lacking. An inefficient immune response allows diseases to develop. Too much, too little, or the wrong immune response, both in timing or intensity, causes immune system disorders. An overactive immune response can lead to the development of "autoimmune diseases," in which antibodies form against the body's own tissues, leading to their destruction or malfunction.

Complications from altered immune responses include:
- Allergy or hypersensitivity
- Anaphylaxis
- Autoimmune disorders
- Graft versus host disease
- Immune and immunodeficiency disorders

Referring to Example 3, inflammation is mediated by a lost balance between pro- and anti-inflammatory immunoregulatory molecules named cytokines. Among them, Interferon-gamma (IFN-y) and Tumor Necrosis-alpha (TNF-α) are proinflammatory cytokines. TNF-α plays a key role in many immune-mediated inflammatory diseases (T Cantaert, D Baeten, PP Tak and LGM van Baarsen, Type I IFN and TNFα cross-regulation in immune-mediated inflammatory disease: basic concepts and clinical relevance. Arthritis Research & Therapy 2010, 12:219; Cytokines and their relationship to the symptoms and outcome of cancer. B Seruga, H Zhang, L Bernstein & IF Tannock, Nature Reviews Cancer 2008, 8, 887). Referring to Example 4, pathogenic influenza virus infection can induce robust cytokine production, excessive inflammatory infiltrates, and virus-induced tissue destruction, that all contribute to morbidity and mortality (JR Teijaroa, KB Walsha, S Ricea, H Rosen and MBA Oldstone, Proc Natl Acad Sci U S A. 2014 Mar 11;111(10):3799-804).

Referring to Example 2, reduced lung function is associated with increased levels of systemic inflammatory markers which may have important pathophysiological and therapeutic implications for subjects with stable Chronic Obstructive Pulmonary Disease (M Miravita, M Calleb, JJ Soler-Cataluñac. Clinical Phenotypes of COPD: Identification, Definition and Implications for Guidelines. Arch Broncopneumol. 2012;48,3:86-98).

In a further aspect, the composition for immunity regulation and/or stimulation of the present invention is provided for its use in a method for enhancing the immune response to antigenic proteins in vaccination protocols.

It is also shown that the composition of the invention may be used for potentiating the immune response to vaccines, and contributes to a better vaccine-mediated prevention of diseases.

"Vaccination" (immunization) is a way to trigger the immune response. Small doses of an antigen, such as dead or weakened live viruses, are given to activate the immune system "memory" (activated B cells and sensitized T cells). Memory allows the body to react quickly and efficiently to future exposures to pathogens carrying the antigen that has been used in the vaccination protocol.

In immunology, adjuvants are components used to potentiate the immune responses to an antigen. An immunologic adjuvant is defined as any substance that acts to accelerate, prolong, or enhance antigen-specific immune responses when used in combination with specific vaccine antigens. Organic and inorganic adjuvants are used in immunology. Two common salts, aluminium phosphate and aluminium hydroxide (Alum), are the most common adjuvants in human vaccines. The precise mechanism of Alum action remains unclear but it can trigger immune cells to promote antibody production.

Referring to Example 5, P27A is an intrinsically unstructured, 104-amino acid long hydrophilic fragment of the Plasmodium falciparum malaria protein PFF0165c, identified and developed by Professor Giampietro Corradin of the University of Lausanne. This blood-stage antigen was found to be the target of human antibodies inhibiting parasite growth in an Antibody-Dependent Cell-mediated Inhibition assay (ADCI). Antibodies acquired by natural exposure were also associated with clinical protection in endemic areas. The vaccine candidate is formulated with aluminium hydroxide (Alum).

Controlled clinical studies have demonstrated a significant aptitude of the composition according to the invention to reinforce the immune system of patients presenting evidence of a down regulated immune system.

Further, the composition according to the present invention shows excellent preventive actions on the reduction of parasitic, respiratory, and intestinal infectious episodes in malnourished children exposed to multiple pathogens in their daily life.

In addition, the composition according to the present invention also shows excellent preventive actions, for example on winter infectious episodes or flu syndrome in 80% of studied patients with recurrent infections in the previous years.

When the composition of the invention is used as therapeutic complement, it was shown that more than 60% of the patients suffering from severe respiratory diseases (severe chronic obstructive bronchopneumopathy disease or Bronchiectasis) showed a 50% diminution of the frequency and duration of infections and of the number and length of hospitalizations.

Besides, the composition of the present invention has evidenced an unexpected strengthening of the natural defence by increasing the CD4 immunity markers and by increasing the weight gain in HIV-infected populations with or without AntiRetroViral (ARV) treatment.

An increase of the efficiency of the ARV treatment to a maximal level was observed.

Surprisingly it was found that the administration of the preparation according to the invention improves the life, as well as the general health and immune status of HIV-infected patients.

The synergy action of the composition for immunity regulation and/or stimulation according to the invention seems to be separated by any other pharmacological action (see the Examples).

In the composition for immunity regulation or and/or stimulation in accordance with the invention, no carrier or pharmaceutically acceptable carrier or suitable excipients are often required to be added. However, suitable excipients may optionally be added.

Examples of suitable excipients of this invention include, but are not limited to, anti-adherents, binders (e.g., macrocrystalline cellulose, gum tragacanth, or gelatin), coatings, disintegrants, fillers, diluents, softeners, emulsifiers, flavouring agents, colouring agents, adjuvants, lubricants, functional agents (e.g., nutrients), viscosity modifiers, bulking agents, glidiants (e.g., colloidal silicon dioxide) surface active agents, osmotic agents, diluents, or any other non-active ingredient, or combinations thereof.

For example, the composition for immunity regulation or prevention of the present invention may include excipient materials selected from the group consisting of calcium carbonate, colouring agents, whiteners, preservatives, and flavours, triacetin, magnesium stearate, sterotes, natural or artificial flavours, essential oils, plant extracts, fruit essences, gelatins, or combinations thereof.

Optionally the preparation of the present invention may include other artificial or natural sweeteners, bulk sweeteners, or combinations thereof. Bulk sweeteners include both caloric and non-caloric compounds. Non-limiting examples of bulk sweeteners include sucrose, dextrose, maltose, dextrin, dried invert sugar, fructose, high fructose corn syrup, levulose, galactose, corn syrup solids, tagatose, polyols (e.g., sorbitol, mannitol, xylitol, lactitol, erythritol, and maltitol), hydrogenated starch hydrolysates, isomalt, trehalose, and combinations thereof.

The active substances in the composition for immunity regulation and/or stimulation according to the invention can change within a wide range.

The composition for immunity regulation and/or stimulation in accordance with the invention may be in the form of a solid formulation, such as capsules or tablets or in the form of a liquid or oil solution. The formulation of the composition described in the present invention can be prepared through any known method in the healthcare food or pharmacological field.

Any method familiar to the skilled in the art can be adopted, that is to say, the composition of the invention is manufactured into a solid formulation, such as capsules and tablets, or a liquid or spray formulation, such as oral liquid and oil solutions to be administered with or without suitable carrier system. The composition of the invention has shown stability over 3 years and more.

In the case of a medicament, the suitable excipient or carrier system can be also a pharmaceutically acceptable excipient.

Suitable carrier systems comprise flow aid, such as micropowder silicon gel and cornstarch, which can enhance the compressibility and can prevent from sticking. For example, hydroxypropyl cellulose, methylcellulose, croscarmellose sodium, various amylum derivatives, silicon dioxide and any other disintegrating agents having the disintegration facilitating function such as tylose, cross-linked sodium carboxymethyl cellulose, various starch derivatives;

In addition, suitable carrier systems may also comprise anti-blushing agent, such as glyceryl behenate, which is helpful for enhancing moisture resistance capability.

Furthermore, suitable carrier systems may also comprise lubricant, such as magnesium stearate, French white and the like, having the lubrication function.

Preferably, the suitable carrier is selected among hydroxypropyl, cellulose, methylcellulose , croscarmellose sodium, various amylum derivatives, silicon dioxide, magnesium stearate, French white, glyceryl behenate and anti-blushing agent.

The present invention further relates to a food preparation, a dietary or food supplement, a nutraceutical, a beverage as well as a medicament comprising the preparation of the present invention. As described above, the medicament may further comprise a pharmaceutically acceptable excipient.

Preferably the medicament, the nutraceutical or dietary supplement of the present invention is administered at a dosage of between 0.1 µg/kg per day to 1 mg/kg per day, preferably between 0.5 µg/kg per day and 100 µg/kg per day.
for the animal or human nutrition.

The composition for immunity regulation and/or stimulation or the medicament is administered orally, parenterally or topically.

If intended for oral administration, the composition of the present invention can be in the form, for example, of a tablet, a caplet, a pill, a hard or soft capsule, a lozenge, a cachet, a dispensable powder, granules, a suspension, an elixir, a dispersion, a liquid, or any other form reasonably adapted for such administration. If intended for parenteral administration, it can be in the form, for example, of a solution for intravenous, intramuscular or subcutaneous injection.

The composition of the invention is effective in preventing and decreasing the severity and number of infectious diseases that may affect subjects.

One object of an invention is to provide a better protection and/or treatment of an individual exposed to a pathogen or organism or microbe, in certain aspects an airborne pathogen or organism or microbe. The composition is administered after or before a subject is at risk or heightened risk of exposure to a potentially pathogenic or pathogenic organism(s).

One object is to use the composition for immunity regulation and/or stimulation in the treatment or prevention of infections episodes or flu syndromes. In particular infectious episodes affecting the lungs and Ear, Nose and Throat (ENT) area generally during the winter months.

The present invention is also particularly effective in the treatment or prevention of severe chronic obstructive bronchopneumopathy disease (COPD) or Bronchiectasis (Chronic dilations of the bronchi).

The present invention is also efficient in decreasing the number and severity of bronchitis cases.

In another use of the invention, gut infections associated with diarrhea are decreased in number and severity, even in geographical areas with poor quality drinking water

The present invention is also efficient in decreasing the number and severity of malaria cases, even in geographical areas where this disease is endemic.

The composition for immunity regulation and/or stimulation is also adapted for use in a method for the potentiating of antibiotics in pulmonary super infections comprising pulmonary, ENT; urinary or skin infections.

Besides the composition for immunity regulation and/or stimulation according to the invention is adapted for use in a method for the lowering of secondary infections due to chronic pathologies.

In a further embodiment the composition for immunity regulation and/or stimulation is the invention is adapted for use in a method for increasing the mucosal immune defence.

The present invention is also particularly effective in the treatment or prevention of chronic obstructive bronchopneumopathy disease (COPD) or Bronchiectasis (Chronic dilation of the bronchi).

Another aspect of the composition for immunity regulation and/or stimulation according to the invention concerns its use in a method for alleviating HIV infection / AIDS disorders. The present invention is used for stimulation of natural immune defences and immune system regeneration in a method for alleviating HIV infection / AIDS disorders.

In another embodiment the composition for immunity regulation and/or stimulation according to the invention is to be used in a method for the regulation of inflammation due to an inappropriate immune response or to infections.

In a further embodiment the composition for immunity regulation and/or stimulation according to the invention is used in a method for enhancing the immune response to antigenic proteins in vaccination protocols.

The foregoing description will be more fully understood with reference to the following Examples. Such Examples, are, however, exemplary of methods of practising the present invention and are not intended to limit the scope of the invention.

### Examples

### Example 1:

### Strengthening of natural defences in HIV-infected patients

Controlled clinical studies were conducted to evaluate the effectiveness of the composition of the invention to reinforce the immune system of patients presenting evidence of a downregulated immune system or of a high susceptibility to infections with no identified cause.

### 1. Strengthening of natural defences in HIV-infected patients - Study TOGO I

A clinical study was conducted with the NGO Espoir Vie TOGO under the coordination of Pr Mireille David, University of Benin (Lome-Togo)

### Purpose of the study

Evaluation in patients non-eligible or eligible for Anti-RetroViral therapy (ARV) of the evolution of the CD4 cell population after 6 months of treatment with the composition of the invention.

### Profile of the study

Two armed (plus or minus ARV) randomized, open study during 6 months.

### Patient inclusion

Population: 100 seropositive persons (PVHIV) split in 2 groups.
Group 1: PVHIV who did not need ARV (in view of their good immune system)
Group 2: PVHIV whose immune and/or clinical status was requiring ARV.

Compliance was not sufficient for 23 patients in the course of the study

### Posology

2 capsules twice a day of the composition of the invention.

### Duration

9 months.

### Patient monitoring

- Weight
- Assessment of CD4 lymphocytes by measurement of blood CD4 count.

### Results:

The positive effect of the composition is evidenced by an increase of weight and CD4 cells in composition-treated patients.

**Table 1: weight and CD4 count in patients treated with the composition of the invention**

| | Number of patients | ARV | Weight gain | CD4 increase |
|---|---|---|---|---|
| 1^{st} group | 31 | no | 5.2kg (+ 9.7%) | 61/mm³ (+37.6%) |
| 2^{nd} group | 46 | yes | 10.1kg (+18.8%) | 208/mm³ (+174.7%) |

### Conclusions

The composition of the invention increases the weight of immunodeficient patients and the CD4 cell counts in both groups (with/without ARV).

The composition of the invention has a very positive effect on the evolution of the immune status and is therefore able to reinforce the immune system of weakened patients.

### 2. Strengthening of natural defence in HIV-infected patients - Study TOGO II

A second clinical study was conducted under the coordination of Pr Mireille David, University of Benin (Lome-Togo). This randomized double-blind clinical study, run in Togo with collaborators from the Pasteur Institute, confirmed the significant efficacy of the composition of the invention on CD4 cell increase observed in the study Togo I.

### Purpose of the study

Further evaluate how the composition of the invention protects AIDS patients by preventing the loss of CD4 cells over time. Evaluation in subjects non-eligible for ARVs, and whose CD4 counts are between 250 and 400 cells per µl, of the evolution of CD4 cell counts at 12 months of the composition of the invention treatment vs. placebo.

### Profile of the study

Double-blind randomized therapeutic trial in subjects not eligible for ARVs, but with T lymphocytes CD4+ counts between 250 and 400 per µl.

### Patient inclusion

132 patients recruited and followed:
- Group 1: composition of the invention, 62 patients.
- Group 2: placebo, 70 patients.

**Table 2: Distribution of patient groups during the study**

| | Treated | Excluded | | Followed | |
|---|---|---|---|---|---|
| | | Size | % | Size | % |
| Composition | 62 | 21 | 33.9 | 41 | 66.1 |
| Placebo | 70 | 25 | 35.7 | 45 | 64.3 |
| Total | 132 | 46 | 34.8 | 86 | 65.2 |

### Posology

2 capsules twice a day of the composition of the invention or placebo.

### Duration

12 months.

### Patient monitoring

Assessment of in T lymphocyte levels by measurement of blood CD4 counts.

### Results

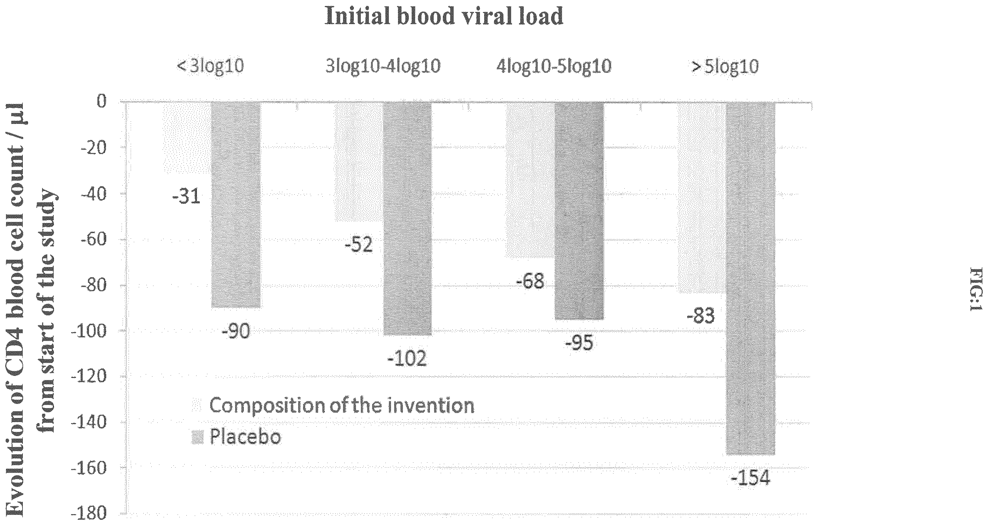

Evolution according to treatment and initial viral charge (viruses/µl). See **figure 1****.**

This figure shows the evolution of the CD4 counts in function of the initial viral charge. This figure clearly demonstrates the antiviral effect of the composition according to the invention in decreasing the viral charge. A high viral charge is a factor of acceleration for CD4 decrease, and thus of progression towards AIDS disease.

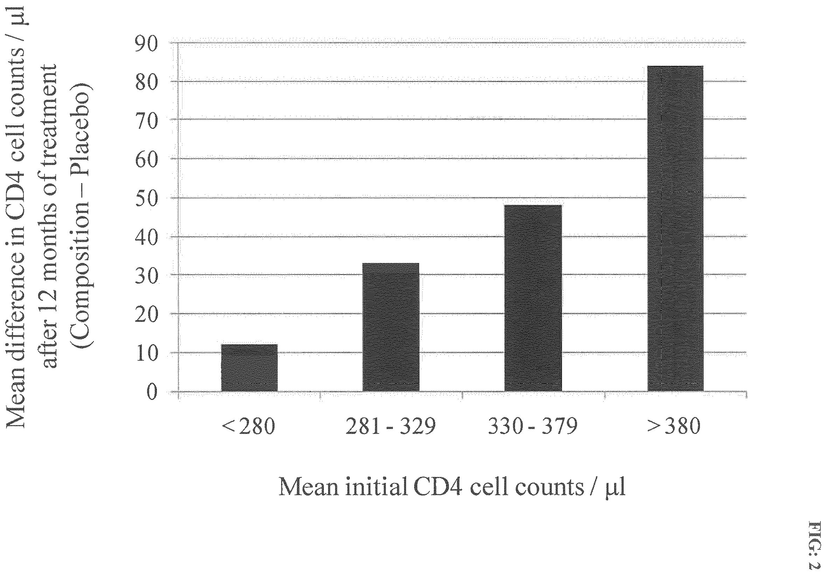

Evolution according to treatment and initial CD4 count (CD4 cells/µl). See **figure 2****.**

This figure shows the evolution of the CD4 counts over time. Each point corresponds to the mean difference in the loss of CD4 cells due to the composition of the invention vs. placebo.
- For patients with an initial CD4 count above 380, the gain attributable to of the composition of the invention is significant at month 3 (M3) and increases with time.
- By contrast, for those whose initial CD4 counts were above 280-379, the gain attributable to of the composition of the invention appears after M9.
- For patients whose initial CD4 was below 280, no statistically significant difference between the group of the composition of the invention and placebo was observed.

### Conclusion

This double-blind study carried out in Togo shows that the composition of the invention protects AIDS patients by preventing the loss of CD4 over time.

This study shows that the composition of the invention protects AIDS patients by preventing the loss of CD4 cells over time. See **figures 1** **and** **2****.**

This initial clinical study carried out in Togo also showed that the composition of the invention protects HIV-infected patients by preventing the loss of CD4 cells over time.
- This effect is more pronounced when the initial blood CD4 count is above 280/µl.
- The composition of the invention decreases by 22.6% the need of ARV therapy after 12 months.

### Example 2:

### Prevention of infectious episodes in lungs and the Ear Nose and Throat (ENT) area

### 1. Prevention of infectious episodes

### Purpose of the study

Evaluation of the effectiveness of the composition of the invention on infectious episodes (including flu syndrome) affecting the lungs and ENT (Ear, Nose & Throat) area during the winter months, and requiring the intervention of a physician.

### Profile of the study

Children and adults, open study during 3 months. Study conducted in France.

### Patient inclusion

60 children (age: 6-15 year-old) and 60 adults (age: 15-70 year-old) presenting weakened natural immunity manifested by recurring winter infections (ENT - repeated bronchitis) and/or flu episodes, indexed during the previous two years in liberal practice.

### Posology

2 capsules of the composition of the invention per day.

### Duration

3 winter months: October - November - December.

### Patient monitoring

- Number of winter broncho-ENT infections
- Number of flu episodes

### Results

Effect of the composition of the invention on winter broncho-ENT infections and flu episodes

### (Figures 3 and 4).

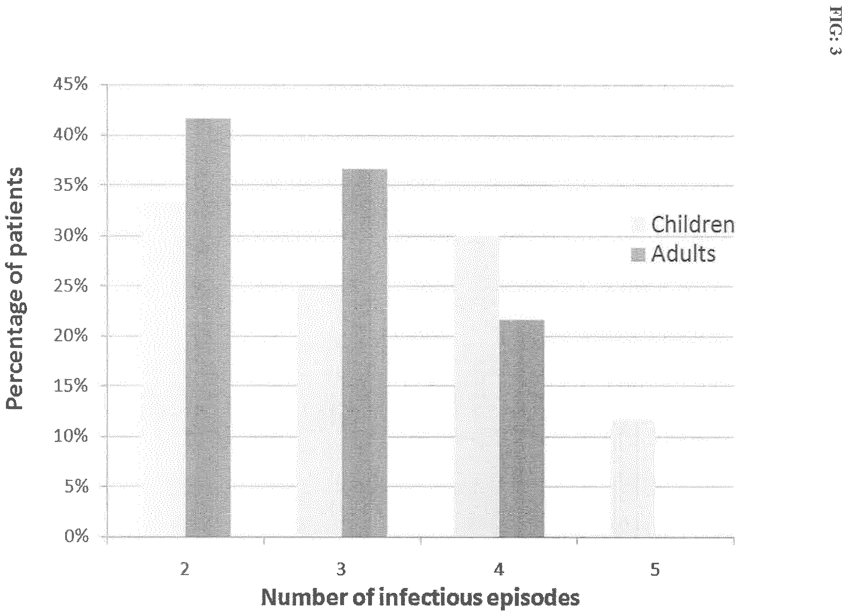

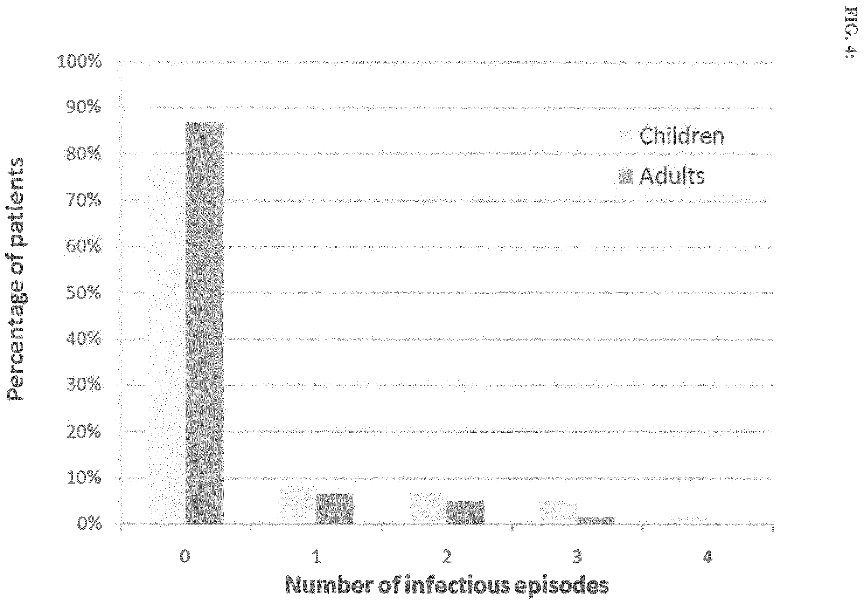

The composition of the invention leads to the disappearance of the infectious episodes during all winter in a range of 78% in children and of 86% in adults, likely by strengthening of the natural immunity **(****figure 4****).** This is a dramatic improvement compared to the infectious episodes having affected these patients in the previous two years **(****figure 3****).**

These figures indicate an increase of natural immunity similar to the one observed with the flu virus vaccine (70% at best).

### Conclusion

The intake of the composition of the invention in a prophylaxis setting allows the elimination of infectious episodes during the winter period in a great majority of cases. 82.5% of the studied population, who in previous years were presenting recurring lung and ENT infections, did not suffer such episodes when taking the composition of the invention.

### 2. Secondary infections of pulmonary chronic pathologies: the prevention and accompaniment of usual therapeutic treatments:

### Study 1 - Ancillary therapy for Chronic Obstructive Pulmonary Disease (COPD)

### Purpose of the study

Evaluate of the effectiveness of the composition of the invention on the frequency of repeated infections in people suffering from severe Chronic Obstructive Pulmonary Disease (COPD).

### Profile of the study

Open study of 3 months. The frequency of infectious episodes was compared to the previous years. The study was conducted in France.

### Patient inclusion

60 adults (49 to 76 year-old, mean age: 66.3) presenting winter infections (repeated bronchitis), indexed during the previous two years and followed in hospital.
- 52 men and 8 women with previous (60) and/or still active (42) nicotinism.
- Forced Expiratory Volume in 1 second (FEV1) between 1250 and 1500ml
- Tiffeneau (ratio FEV1 / Forced Vital Capacity) < 55%
- Neither cardiac repercussion, nor repercussion on arterial gazometry.
- Medical treatment: bronchodilators - drainage physiotherapy - vaccination (flu, pneumococci), antibiotherapy ± corticoids in short cure.

### Posology

3 capsules of the composition of the invention per day during 3 months in the autumn-winter period by patients who used to take Biostim the previous years at the same period, and who still were under anti-flu and anti-pneumococci prevention treatment.

### Patient monitoring

- Quantity and purulence of expectorations
- Frequency and duration of infections
- Recovery with or without antibiotics
- Frequency and duration of hospitalizations

### Results

### - Frequency of infections:

**Table 3: Frequency of infections in COPD patients**

| Number of patients | Before composition of the invention | With composition of the invention | % difference |
|---|---|---|---|
| 12 | 5 | 3 | |
| 13 | 4 | 4 | |
| 14 | 4 | 2 | |
| Total | 13 | 9 | 30% |

A decrease in the mean number of infections was observed in 67% of patients (26 out of 39 patients) with COPD.

### - Frequency of infections (tuberculosis history)

**Table 4: frequency of infections in COPD patients with tuberculosis history**

| Number of patients | Before composition of the invention | With composition of the invention | % difference |
|---|---|---|---|
| 10 | 5 | 3 | |
| 11 | 3 | 3 | |
| Total | 8 | 6 | 25% |

A decrease in the mean number of infections was observed in 50% of patients (10 out of 21 patients) with COPD associated with severe history of tuberculosis

### - Average number of hospitalizations

**Table 5: Number of hospitalizations and length of stay in hospital of COPD patients**

| Number of patients | Number of hospitalizations | | Number of days in hospital | |
|---|---|---|---|---|
| Base 53 | Before | After | Before | After |
| 41 | 2 | 1 | 10 | 6 |

A decrease in the mean number of hospitalizations (from 2 to 1) and in the mean number of days in hospital (from 10 to 6) was observed in 68 % of the cases (41 out of 53 patients).

### - Diminution of the quantity of expectorations

**Table 6: Quantity of expectorations in COPD patients**

| Number of patients | Before composition of the invention | After composition of the invention | % difference |
|---|---|---|---|
| 18 | 35 ml | 15 ml | |
| 27 | 20 ml | < 5 ml | |
| Total: 45 | 55 ml | 20 ml | 64% |

A significant diminution of the mean quantity of expectorations 72 hours after taking the composition of the invention was observed in 40 % of the cases (18 out of 45 patients), and a quasi-elimination of the volume of expectorations (< 5 ml) was observed in 60% of the cases (27 out of 45 patients).

### - Antibiotherapy

No systematic antibiotherapy as compared to the two previous years in 64 % of the cases (25 out of 39 patients).

### Conclusion

The intake of composition of the invention in prevention in patients suffering from COPD enables to reduce:
- The quantity and purulence of expectorations
- The frequency of infections
- The systematic use of antibiotherapy
- The number and length of hospitalizations

For the majority of patients suffering from COPD, the composition of the invention is effective in alleviating or preventing COPD signs as an adjuvant to usual treatments.

### Study 2 - Ancillary therapy for Bronchiectasis (chronic dilatations of the bronchi) Purpose of the study

Evaluation of the effectiveness of the composition of the invention on the evolution frequency of repeated infections in people suffering from bronchiectasis (chronic dilatation of the bronchi). Study conducted in France.

### Profile of the study

Open study during 3 months. Comparison with the frequency of episodes in previous years.

### Patient inclusion

27 adults (48 to 58 year-old, average age: 53.6) presenting repeated winter infections, indexed during the previous two years and followed in the hospital.
- Female predominance.
- No previous nicotinism.
- Forced Expiratory Volume in 1 second (FEV1) between 1250 and 1700ml
- Tiffeneau (ratio FEV1 / Forced Vital Capacity) < 60%
- Vital capacity (VC) / Total Pulmonary capacity (VCt) > 80%
- No cardiac repercussion, nor repercussion on arterial gazometry.
- Medical treatment: drainage physiotherapy twice a day - vaccination (flu, pneumococci), antibiotherapy ± bronchodilators ± corticoids in short cure.

### Posology

3 capsules of the composition of the invention per day.

### Patient monitoring

- Quantity and purulence of expectorations
- Frequency and duration of infections
- Recovery with or without antibiotics
- Frequency and duration of hospitalizations

### Results

### - Frequency of infections

**Table 7: frequency of infections in bronchiectasis patients**

| Number of patients | Before composition of the invention | After composition of the invention | % difference |
|---|---|---|---|
| 8 | 7 | 4 | |
| 12 | 6 | 3 | |
| 7 | 5 | 5 | |
| Total: 27 | 18 | 12 | 33% |

In 74 % (20 out of 27 patients) of the cases, a decrease in the mean number of infections in the period from October to March was observed.

### - Average number of hospitalizations

**Table 8: Number of hospitalizations and length of stay in hospital of bronchiectasis patients**

| Number of patients | Number of hospitalizations | | Number of days in hospital | |
|---|---|---|---|---|
| Base 27 | Before | After | Before | After |
| 18 | 2 | 1 | 15 | 8 |

In 66 % of the cases (18 out of 27 patients), a diminution in the mean number of hospitalizations (from 2 to 1) and mean hospitalization days (from 15 to 8) was recorded.

### - Antibiotherapy

In 52 % of the cases (14 out of 27 patients), there was no need for systematic antibiotherapy as compared to the two previous years.

### - Volume of expectorations

No modification of the mean volume of expectorations was observed.

### - Asthenia

Diminution of asthenia in 64% of the cases.

### Conclusion

The intake of the composition of the invention in prevention in patients suffering from Bronchiectasis enables to reduce:
- The frequency of infections
- The systematic use of antibiotherapy
- The number and length of hospitalizations
- The occurrence of asthenia

For the majority of patients suffering from COPD, the composition of the invention is effective as an adjuvant to the usual treatments.

### 3. Reduction of number of infectious episodes in cohorts of children with moderate malnutrition with multiple infectious episodes

### Purpose of the study

Demonstrate the efficacy of the composition on the prevalence of infections in cohorts of children with moderate malnutrition, thus subject to multiple infectious episodes.

### Study conducted in Rwanda

### Profile of the study

Two armed randomized, double blind study during 12 months.

### Patient inclusion

- Inclusion of 220 children aged 1 to 5 years, with moderate malnutrition and repeated infections in the year preceding the study
- One arm of 110 children
- One arm of 110 children

### Posology

2 capsules of the composition of the invention per day.

### Duration

### 12 months

### Patient monitoring

Children were monitored each month by a doctor and a nurse in three health centers in the district of Bugesera in Rwanda

### Main objective

The main objective was to compare infectious episodes in pediatric populations of 12 to 60 months old children over a period of 12 months depending on whether they received the composition of the invention or Placebo.

### Results

### - Number of infectious episodes

The pleiotropic protective effect of the composition of the invention was evidenced when the placebo- and composition-treated children were classified according to the number of infections:

**Table 9: Number of children of both the placebo and the composition-treated groups according to the number of infectious episodes recorded during the study.**

| Nb of infectious episodes | **Composition** | **Placebo** | **Total** |
|---|---|---|---|
| 0 | 1 | 3 | 4 |
| 1 | 11 | 5 | 16 |
| 2 | 18 | 11 | 29 |
| 3 | 20 | 15 | 35 |
| 4 | 14 | 16 | 30 |
| 5 | 16 | 15 | 31 |
| 6 | 11 | 10 | 21 |
| 7 | 9 | 17 | 26 |
| 8 | 3 | 7 | 10 |
| 9 | 1 | 5 | 6 |
| 10 | 1 | 0 | 1 |
| 11 | 0 | 2 | 2 |
| 12 | 2 | 1 | 3 |
| 13 | 1 | 2 | 3 |
| 14 | 0 | 0 | 0 |
| 15 | 0 | 0 | 0 |
| 16 | 1 | 0 | 1 |
| Median | 2.0 | 5.0 | |

The median is the numerical value separating the higher half of a data sample, a population, or a probability distribution, from the lower half. The median is the "middle" value in the list of numbers. The group of children treated with the composition of the invention showed a median value of 2, while the mean value of the placebo group was 5. This indicates that the composition shifts to lower numbers the middle of the infectious episodes ranking.

The protective effect of the composition of the invention was evidenced when the total number of infectious episodes was recorded in the placebo and composition-treated groups:

**Table 10: Number of infectious episodes in the placebo and composition-treated children:**

| Diagnostic | Placebo | Composition | % difference | Total |
|---|---|---|---|---|
| Flu | 161 | 126 | 21.2 | 287 |
| Diarrhea (intestinal infections) | 112 | 96 | 14.3 | 208 |
| Malaria | 112 | 88 | 21.4 | 200 |
| Bronchitis with fever | 29 | 25 | 13.8 | 54 |
| Bronchitis (< 3 y.o.) | 21 | 10 | 52.0 | 31 |
| Fever alone | 10 | 1 | 90 | 11 |
| Urinary tract infection | 2 | 0 | 100 | 2 |
| Parotiditis | 1 | 0 | 100 | 1 |
| Total | 448 | 346 | 22.8 | 794 |

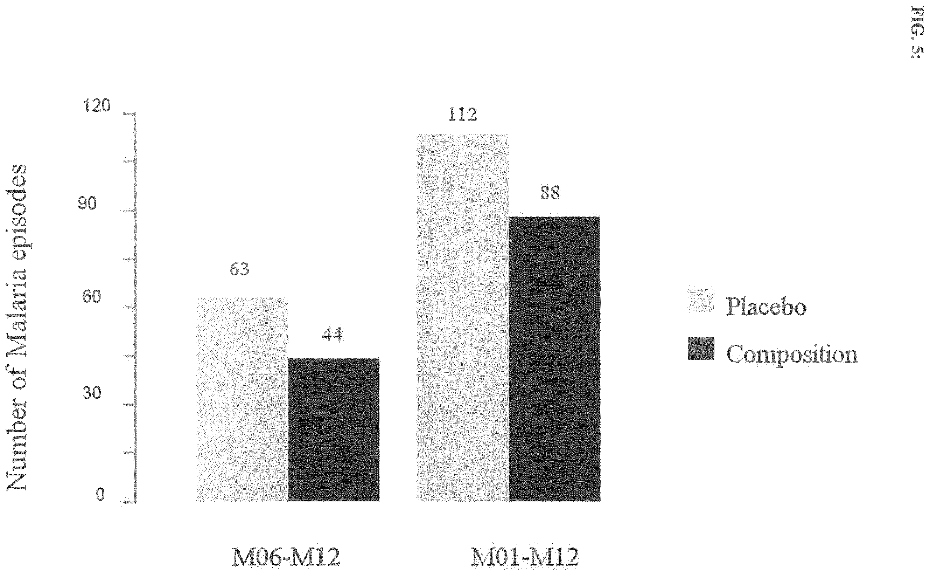

The composition of the invention significantly reduces the number of Malaria episodes: - 21.4 % between months 1 and 12 (p<0.001) and up to - 30 % between months 6 to 12 **(****Figure 5****)**

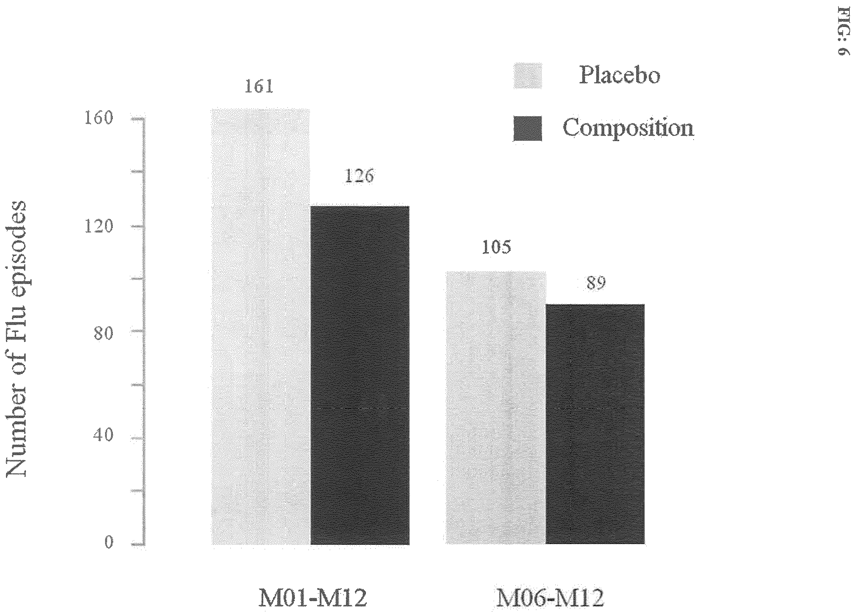

The composition of the invention significantly reduces the number of Flu episodes: - 21.2 % between months 1 to 12 (p=0.004, **Figure 6****)** and -25% between months 6 and 12.

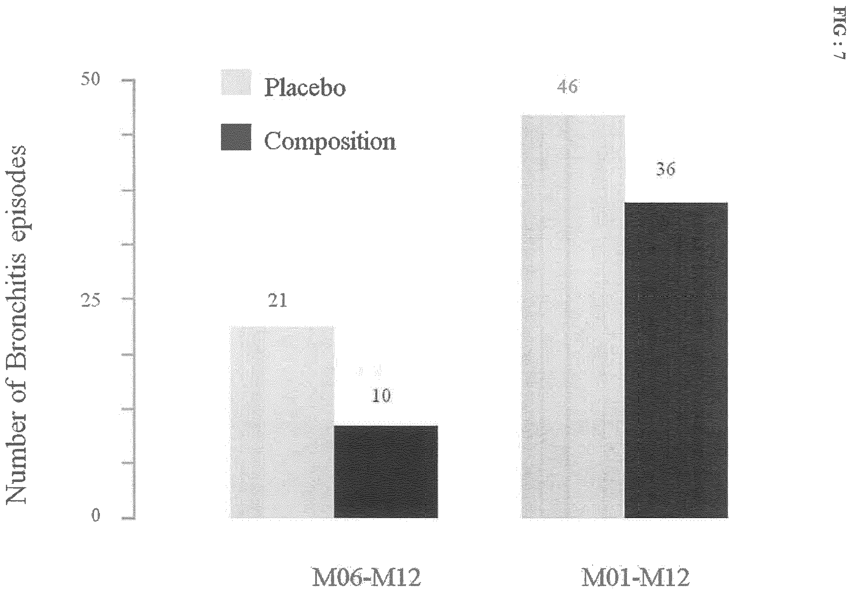

The composition of the invention significantly reduces the number of Bronchitis episodes in children below 36 months old: - 22 % between months 1 to 12 and up to - 52 % between months 6 to 12 (p=0.003, **Figure 7****)**

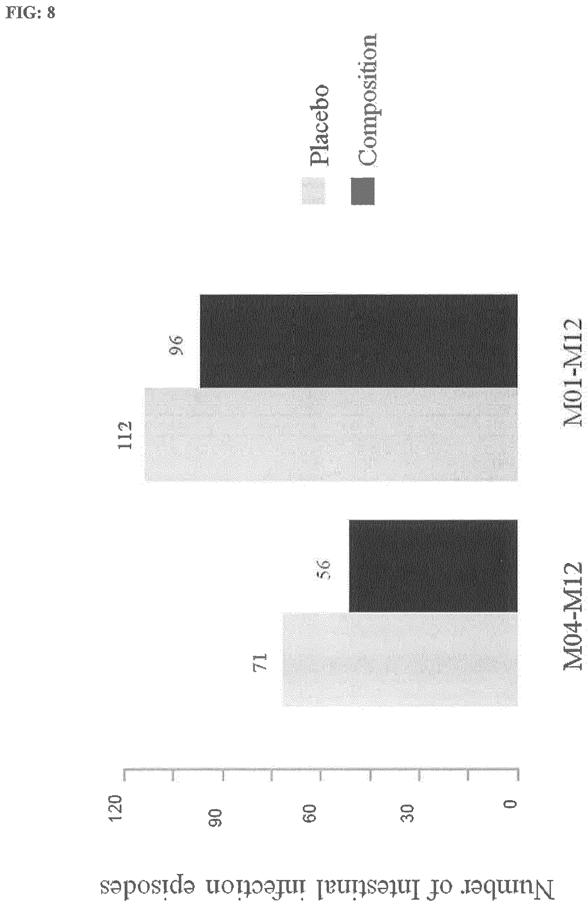

The composition of the invention significantly reduces the number of Intestinal infection episodes evidenced by diarrhea: - 14.3% between months 1 and 12 and - 21% between months 4 to 12 (p=0.039, **Figure 8****).**

The protective effect of the composition was also evidenced when the number of mucosal and skin infections was recorded:

**Table 11: Number of skin and mucosal infections**

| Diagnostic | Placebo | Composition | % difference | Total |
|---|---|---|---|---|
| Conjunctivitis | 17 | 9 | 47.1 | 26 |
| Herpes | 5 | 1 | 80 | 6 |
| Impétigo | 5 | 0 | 100 | 5 |
| Mycosis | 2 | 0 | 100 | 2 |
| Total | 29 | 10 | 65.6 | 39 |

The composition of the invention significantly reduced the number of mucosal and skin infections.

The composition of the invention significantly reduced the number of severe illnesses:
- The protective effect of the composition was also evidenced when the number of severe cases of malaria, associated with anemia, convulsion or gastroenteritis, was recorded:

**Table 12: Number of severe cases of malaria episodes**

| Associated medical issue | Placebo | Composition | % difference |
|---|---|---|---|
| Anemia | 2 | 0 | 100 |
| Convulsion | 5 | 3 | 40 |
| Gastroenteritis | 2 | 0 | 100 |
| Total | 9 | 3 | 66 |

- The protective effect of the composition was also evidenced when the number of severe cases of diarrhea, associated with fever or with more than 8 per day were recorded:

**Table 13: Number of severe cases of diarrhea**

| Associated medical issue | Placebo | Composition | % difference |
|---|---|---|---|
| Fever | 29 | 20 | 31 |
| >= 8 stool / day | 15 | 12 | 20 |
| Total | 44 | 32 | 27.3 |

- The protective effect of the composition was also evidenced when the number of severe cases of bronchitis, associated with bronchopneumonia, were recorded:

**Table 14: Number of severe cases of bronchitis**

| Associated medical issue | Placebo | Composition | % difference |
|---|---|---|---|
| Bronchopneumonia | 9 | 6 | 33 |

### Conclusions:

The intake of the composition of the invention reduces significantly the number of infectious episodes.

The intake of the composition of the invention is effective in the prevention of the most common infections: flu, bronchitis, intestinal infections.

The intake of the composition of the invention is effective in the prevention of malaria episodes.

The intake of the composition of the invention is effective in the prevention of severe illnesses.

### General conclusions of the effect of the composition of the invention for examples 1 & 2: The composition of the inventions enables a strengthening of natural immune defence:

- Increase in CD4 immunity markers and weight gain in HIV-infected and AIDS patients with or without AntiRetroViral therapy (ARV).
- Increase of the efficiency of the ARV to a maximal level.
- Decrease by 22.6% the necessity of being treated with ARVs after 12 months correlated with the risk of opportunistic infections. This means that treatment of 1000 patients with the composition of the invention prevents the need of 226 ARV treatments over 12 months to maintain the initial CD4 count above 250/µl.

### Prevention setting:

The composition of the invention decreases the occurrence and severity of all diseases studied:
- Disappearance in a population of healthy children and adults of winter infectious episodes or flu syndrome in 80% of the studied cases.
- Prevention of flu syndrome of 21% in comparison with the placebo in a population of moderate malnutrition children
- Prevention of bronchitis episodes of 52% in comparison with the placebo in a population of moderate malnutrition children below 36 months
- Prevention of intestinal infections episodes of 21% in comparison with the placebo in a population of moderate malnutrition children
- Decrease of the frequency of infections in populations with broncho-pulmonary diseases.
- Prevention of skin and mucosal infections.

### Therapeutic complement:

More than 60% of the patients suffering from severe respiratory diseases (severe chronic obstructive bronchopneumopathy disease or Bronchiectasis) showed a diminution of the frequency and duration of infections and of the number and days of hospitalizations when treated with the composition of the invention, conferring an increased quality of life.

The composition of the invention is efficient in the prevention and/or the treatment of the most frequent diseases, comprising:
- Bacterial infections, associated with gram+ or gram- bacteria, of the Ear Nose and Throat area, of the lungs, of the gut and intestinal tract, of the bladder and of the skin and other mucosal areas of the body.
- Viral infections of the Ear Nose and Throat area, of the lungs, of the gut and intestinal tract, of the bladder and of the skin and other mucosal areas of the body.
- Fungal infections
- Parasitic infections, including malaria infection

As the effect of the composition of the invention in the prevention and the treatment of these diseases occurs by a strengthening and a better regulation of the immune system, making such immune system more effective against all external aggressions due to pathogens or diseases, those skilled in the art will appreciate that the effect of the composition of the invention is not limited to the diseases investigated in these studies.

As the intake of the composition of the invention decreased the frequency and severity of the wide spectrum of diseases investigated in these studies, those skilled in the art will appreciate that the composition is effective for protection against pathogens or immune disorders infecting or affecting a subject through various routes, such as:
- droplet contact - coughing or sneezing on another person;
- direct physical contact - touching an infected person, including sexual contact;
- indirect physical contact - usually by touching soil contamination or a contaminated surface
- airborne transmission - involving mucosal lung and digestive tract contact;
- fecal-oral transmission - usually from contaminated food or water sources;
- indirect transmission such as infection by another organism, either a vector (e.g. a mosquito) or an intermediate host.

### Example 3:

### Anti-inflammatory effects of the composition of the invention

In vitro studies were conducted to evaluate the anti-inflammatory effect of the composition on human peripheral blood mononuclear cells (PBMC) stimulated by the association of anti-CD3 and anti-CD28 antibodies (CD3+CD28) that mimics antigenic presentation on T cells.

### 1. Measurement of Interferon-γ (IFN-γ) and Tumor Necrosis Factor-α (TNF-α) cytokine release in a T cell activation model of peripheral blood mononuclear cells (PBMC)

In vitro studies were conducted to evaluate the anti-inflammatory effect of the composition on peripheral blood mononuclear cells (PBMC).

The effect of the composition was evaluated on the release of IFN-y and TNF-α cytokines by activated PBMCs. PBMCs were stimulated by the association of anti-CD3 and anti-CD28 antibodies, a stimulatory regime that mimics antigen-triggered T cell activation.

Three independent experiments were performed in parallel on 3 different donors.

Peripheral Blood Mononuclear Cells (PBMC) are blood cells that are a critical component in the immune system to fight infection and adapt to intruders. They consist in lymphocytes, monocytes and macrophages. The human lymphocyte population consists of T cells (CD4 and CD8 positive ~75%), B cells and NK cells (~25% combined).

Interferon-gamma (IFN-y) is a pro-inflammatory cytokine produced by activated T cells. Tumor Necrosis Factor-alpha (TNF-α) is a pivotal pro-inflammatory cytokine produced by a variety of cells, such as macrophages, activated T cells, natural killer cells and mast cells. IFN-y and TNF-α both participate in cell-mediated immunity.

### Study 1:

PBMC were extracted from whole human blood using ficoll, a hydrophilic polysaccharide that separates layers of blood, which separates the blood into a top layer of plasma, followed by a layer of PBMCs and a bottom fraction of polymorphonuclear cells (such as neutrophils and eosinophils) and erythrocytes. The polymorphonuclear cells were further isolated by lysing the red blood cells.

PBMC were activated for 2 days in vitro with a combination of antibodies anti-CD3 and anti-CD28 (CD3: 10 µg/ml, CD28: 3 µg/ml).

PBMC were incubated in the absence or the presence of the composition of the invention at 4 different concentrations: 0.04; 0.6; 8.8, and 133 ng / ml.

Cyclosporin A was used as positive control at a concentration of 1µM.

IFN-y and TNF-α were quantified in cell culture supernatants with an ELISA (Diaclone) according to manufacturer's instructions.

### Results:

**Table 15: Inhibition of IFN-y secretion by the composition of the invention**

| Composition of the invention (ng/ml) | Donor 1 | Donor 2 | Donor 3 | Average inhibition (%) | Standard Deviation |
|---|---|---|---|---|---|
| | % inhibition of IFN-γ secretion | | | | |
| 0.04 | 27 | 1 | 6 | 11 | 14 |
| 0.6 | 1 | 1 | 4 | 2 | 2 |
| 8.8 | 18 | 10 | 11 | 13 | 4 |
| 133 | 78 | 26 | 64 | 56 | 26 |
| Cyclosporin A | 97 | 95 | 93 | 95 | 2 |

**Table 16: Inhibition of TNF-α secretion by the composition of the invention**

| Composition of the invention (ng/ml) | Donor 1 | Donor 2 | Donor 3 | Average inhibition (%) | Standard Deviation |
|---|---|---|---|---|---|
| | % inhibition of TNF-α secretion | | | | |
| 0.04 | 1 | 1 | 23 | 8 | 12 |
| 0.6 | 3 | 25 | 40 | 23 | 19 |
| 8.8 | 11 | 29 | 24 | 21 | 9 |
| 133 | 10 | 46 | 72 | 43 | 31 |
| Cyclosporin A | 88 | 91 | 92 | 91 | 2 |

The composition induced a significant inhibitory effect on IFN-y release by activated PBMC, reaching up to 78% inhibition (donor 1) at 133 ng /ml.

The composition induced a significant inhibitory effect on TNF-α release by activated PBMC, reaching up to 40% (donor 3) inhibition at 0.6 ng/ml and 72% (donor 3) at 133 ng/ml. The reference cyclosporin A, tested at 1 µM, significantly inhibited IFN-y and TNF-α release by activated PBMC. These results were expected and validated this assay.

### Conclusion

Under the experimental conditions of this assay, the composition induced a significant inhibition of IFN-γ and TNF-α cytokine release by activated PBMC. Taking into account the experimental variations inherent to the use of biological material, the composition showed significant anti-inflammatory activity in a T cell antigenic stimulation model and this effect was observed for concentrations as low as 0.6.

### Study 2

PBMC from one donor were extracted and activated for 2 days in vitro with a combination of antibodies anti-CD3 and anti-CD28 (CD3: 10 µg/ml, CD28: 3 µg/ml) as in study 1.

PBMC were incubated in the absence or the presence of the composition of the invention at 2 different concentrations: 0.6 and 8.8 ng / ml.

Cyclosporin A was used as positive control at a concentration of 1µM.

IFN-γ and TNF-α were quantified in cell culture supernatants with an ELISA (Diaclone) according to manufacturer's instructions.

### Results:

**Table 17: Inhibition of IFN-γ secretion by the composition of the invention**

| Composition of the invention (ng/ml) | % inhibition of IFN-γ secretion | Standard Deviation |
|---|---|---|
| 0.6 | 62 | 3 |
| 8.8 | 28 | 2 |
| Cyclosporin A | 90 | 0 |

**Table 18: Inhibition of TNF-α secretion by the composition of the invention**

| Composition of the invention (ng/ml) | % inhibition of TNF-α secretion | Standard Deviation |
|---|---|---|
| 0.6 | 44 | 1 |
| 8.8 | 27 | 9 |
| Cyclosporin A | 91 | 1 |

The composition induced a significant inhibitory effect on IFN-γ release by activated PBMC, reaching up to 62% inhibition at 0.6 ng /ml.

The composition induced a significant inhibitory effect on TNF-α release by activated PBMC, reaching up to 44% inhibition at 0.6 ng/ml.

The reference cyclosporin A, tested at 1 µM, significantly inhibited IFN-γ and TNF-α release by activated PBMC. These results were expected and validated this assay.

### Study 3

To rule out a cytotoxic effect of the composition of the invention, Jurkat cells were incubated for 2 days with various concentrations of the composition of the invention and the cell viability was quantified by MTT reduction assay.

The MTT assay is a colorimetric assay for assessing cell viability. NAD(P)H-dependent cellular oxidoreductase enzymes may, under defined conditions, reflect the number of viable cells present. These enzymes are capable of reducing the tetrazolium dye MTT 3-(4,5-dimethylthiazol-2-yl)-2,5-diphenyltetrazolium bromide to its insoluble formazan, which has a purple color. Tetrazolium dye assays can be used to measure cytotoxicity (loss of viable cells) or cytostatic activity (shift from proliferative to resting status).

### Results

**Table 19: Effect of the composition of the invention on Jurkat cell viability**

| Composition of the invention (ng/ml) | Cell viability (% of the control) | Standard deviation |
|---|---|---|
| 0 | 100 | 2 |
| 0.000128 | 94 | 4 |
| 0.00064 | 104 | 4 |
| 0.0032 | 102 | 4 |
| 0.016 | 100 | 3 |
| 0.08 | 104 | 3 |
| 0.4 | 98 | 3 |
| 2 | 104 | 4 |
| 10 | 97 | 4 |

### Conclusion

The composition of the invention showed no cytotoxicity in vitro under the conditions of the assay as evidenced by the lack of effect on cell viability at all concentrations tested.

### Example 4

### Study of the efficacy of oral administration of the composition of the invention in a mouse model of influenza A infection.

The experiment tested the effect of the composition on the infection of susceptible mice by the Influenza virus.

### Experimental protocol:

C57BL/6 female mice (8 mice / group) at 8 weeks of age were fed for 3 weeks with chow containing or not the composition of the invention at 10, 100 or 800 ng / day / mouse. Mice were infected intranasally with 40 µl of saline solution containing 300 PFU (plaque forming Units) of Influenza A virus (H3N2 Scotland/20/74) under a mixture of ketamine-xylazine (1 and 0.2 mg per mouse, respectively) anesthesia.

Codes used for treatments were as follows:
Vehicle = IMT-A
10 ng composition / day / mouse = IMT-B or IMT10
100 ng composition / day / mouse = IMT-C or IMT100
800 ng composition / day / mouse = IMT-D or IMT800

At day 3 post-infection, lungs and bronchoalveolar fluid (BALF) were sampled and analyzed. BALF was analyzed for the presence of cells, in particular neutrophils, macrophages and lymphocytes.

Lung tissue was homogenized, RNA was extracted and viral load was quantified using the Polymerase chain reaction technique. This expression denotes the number of viral DNA copies, so the viral load.

Groups of mice were as follows:

| Group number | Infection | IMT-A | Tamiflu | IMT-B (IMT10) | IMT-C (IMT100) | IMT-D (IMT800) |
|---|---|---|---|---|---|---|
| 1 | - | | | | | |
| 2 | + | + | | | | |
| 3 | + | | + | | | |
| 4 | + | | | + | | |
| 5 | + | | | | + | |
| 6 | + | | | | | + |

The + symbol refers to the treatment indicated at the top of the column received by the group number of mice indicated in the left column of the table.

### Results:

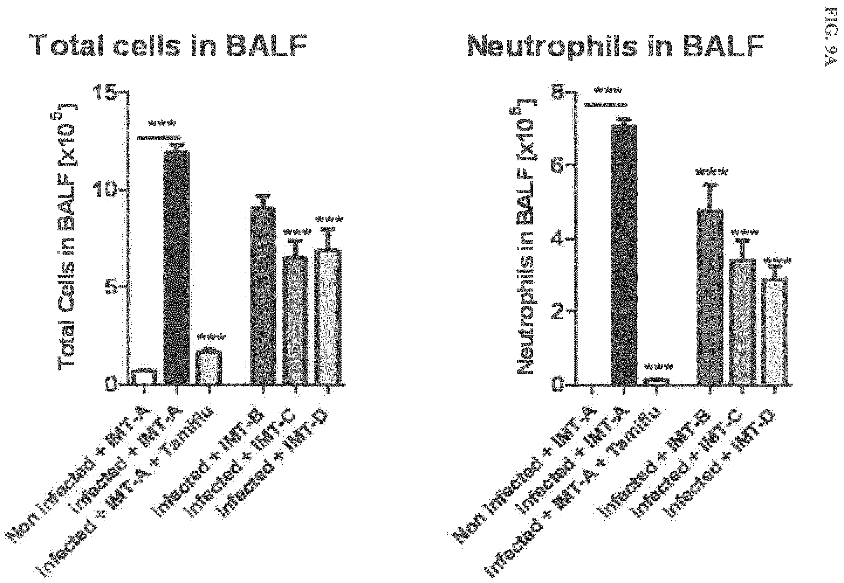

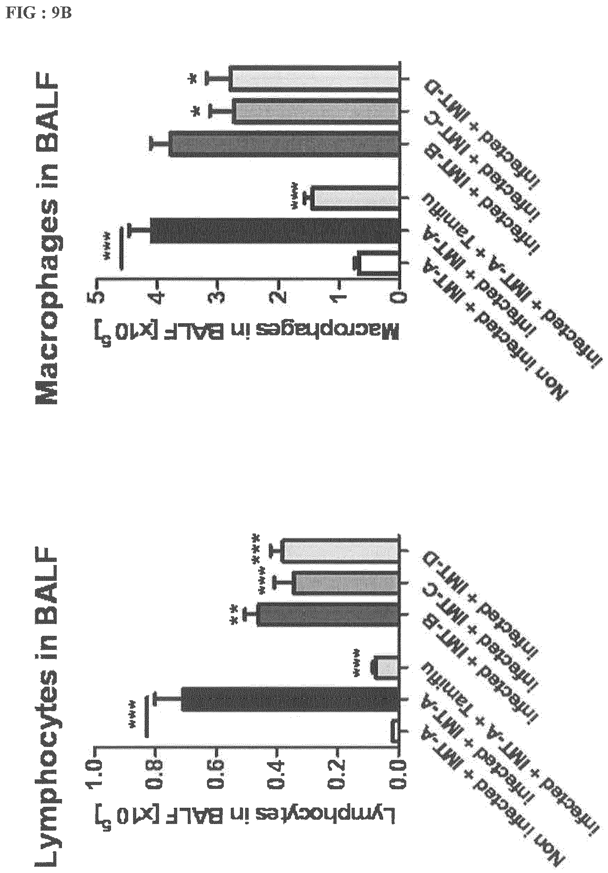

**Figure 9A** **and** **Figure 9B****:** effect of the composition on recruitment of immune cells in the bronchoalveolar lavage fluid (BALF) of mice infected with Influenza Virus A.

The bronchoalveolar fluid of mice was analyzed cytologically for the presence of infiltrating cells. Total cells and neutrophils (Figure 9A) as well as macrophages and lymphocytes (Figure 9B) were quantified.

Treatment with 10, 100 and 800 ng / day of the composition significantly reduced the number of total cells, of neutrophils, of macrophages and of lymphocytes in the bronchoalveolar fluid of influenza-infected mice.

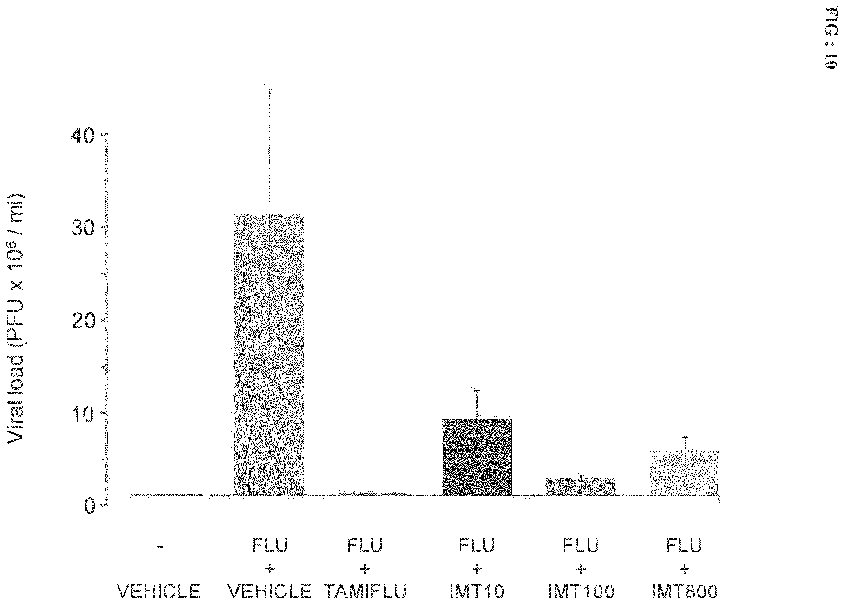

**Figure 10****:** effect of the composition on the Influenza viral load in the lungs of mice infected with Influenza Virus A.

The lungs of mice were homogenized, RNA was extracted and the number of viral copies was quantified using a quantitative polymerase chain reaction protocol.

Treatment with 100 and 800 ng / day of the composition significantly reduced the Influenza viral load in the lungs of influenza-infected mice.

### Conclusions:

Under the experimental conditions of this study, significant anti-inflammatory activity was shown for the composition at all concentrations tested (10, 100 and 800 ng / mouse / day). Indeed, under these conditions, the composition significantly decreased the number of infiltrating cells, including neutrophils, macrophages and lymphocytes in the lungs of infected mice.

Under the experimental conditions of this study, significant protection of mice against Influenza infection was shown for the composition at all concentrations tested (10, 100 and 800 ng / mouse / day). Indeed, under these conditions, the composition significantly decreased the viral load in the lungs of infected mice.

### Example 5:

### Study of the efficacy of oral administration of the composition of the invention in a mouse model of vaccination protocol with a malaria antigenic protein.

### Purpose:

Test the capacity of the composition of the invention to enhance the immune response to an antigenic protein in a vaccination protocol in mice.

### Experimental protocol:

Mice (5 mice per group) were fed with control chow or chow containing the composition of the invention (corresponding to a daily intake of 800 ng / mouse) for 21 days. Mice were then immunized a first time with 20 µg of the protein P27A of the plasmodium falciparum parasite using Alum as an adjuvant, then two times at 21 days interval with 20 µg of the protein P27A of the plasmodium falciparum parasite using Alum as an adjuvant. Blood was drawn 11 days after the last immunization and antibody titers specific for the P27A proteins were quantified by Enzyme-linked immunosorbent assay (ELISA).

### Results:

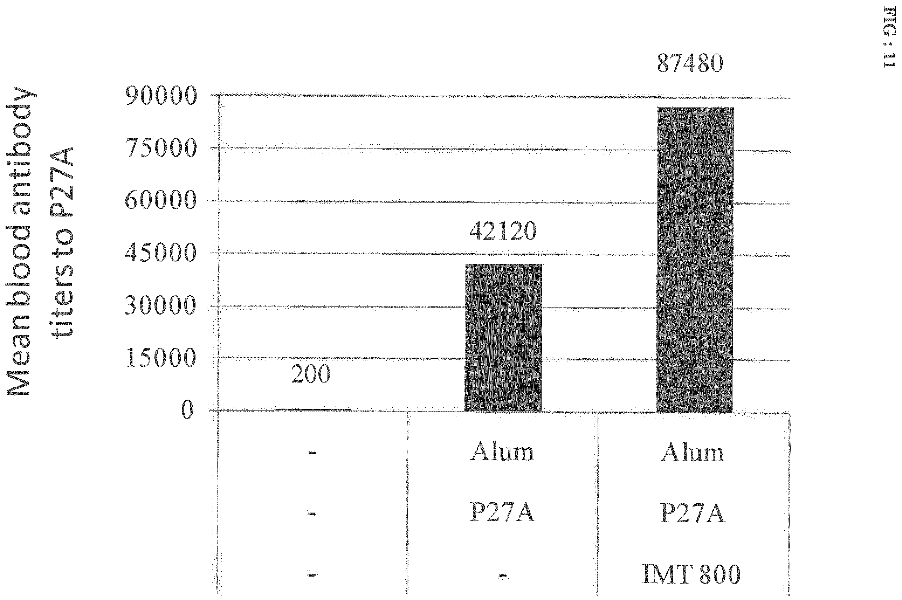

Antibody titers to the P27A protein were augmented more than two-fold in the blood of mice treated with the composition of the invention (**Figure 11**).

### Conclusions:

The composition of the invention is effective in enhancing the immune response to antigens in vaccination protocols.

Aluminum adjuvants are adjuvants for vaccines that confer protection by inducing antibodies via the induction of a type 2 immune response. The composition of the invention enhances this adjuvant effect by stimulating the production of an increased number of antibodies specific for the P27A antigen. This effect may be mediated by increasing the adjuvant effect of the Alum or by rendering the P27A antigen more immunogenic by itself, both mechanisms contribute to an increased antibody production.

Alum is the most commonly used adjuvant in human vaccination. It is found in numerous vaccines, including diphtheria-tetanus-pertussis, human papillomavirus and hepatitis vaccines. Therefore, the composition of the invention may be used for potentiating the immune response to numerous vaccines, and contribute to a better vaccine-mediated prevention of multiple diseases.

### Example 6:

**Table 20: Example of preparation of the formulation for a daily intake / GDA (Guideline Daily Amounts):**

| Formula of the composition of the invention **for one dosis/day** | | |
|---|---|---|
| **Latin name** | **Official English name** | **Daily intake** |
| *Magnesium* | Magnesium | 0.050 to 5 µg |
| *Ferrum* | Iron | 0.050 to 5 µg |
| *Zincum* | Zinc | 0.050 to 5 µg |
| *Thymus vulgaris* | Common thyme | 0.030 to 3 µg |
| *Cicer arietinum* | Chickpea | 0.030 to 3 µg |
| *Ervum lens (Lens culinaris)* | Lentil | 0.030 to 3 µg |
| *Ribes nigrum* | Blackcurrant oil | 0.035 to 3.5 µg |
| *Elaeis guineensis* | Palm oil | 0.035 to 3.5 µg |
| *Fucus vesiculosus* | Fucus | 0.030 to 3 µg |
| *Undaria pinnatifida* | Wakame | 0.030 to 3 µg |
| *Palmaria palmata* | Dulse | 0.030 to 3 µg |
| *Lentinula edodes* | Shiitake | 0.035 to 3.5 µg |
| *Grifola frondosa* | Maitake | 0.035 to 3.5 µg |
| *Porphyra umbilicalis* | Nori | 0.030 to 3 µg |
| Retinol | Vitamin A | 0.040 to 4 µg |
| Thiamine HCl | Vitamin B1 | 0.040 to 4 µg |
| Riboflavine | Vitamin B2 | 0.040 to 4 µg |
| Nicotinamide | Vitamin B3 | 0.040 to 4 µg |
| Calcium pantothenate | Vitamin B5 | 0.040 to 4 µg |
| Pyridoxine HCl | Vitamin B6 | 0.040 to 4 µg |
| Folic acid | Vitamin B9 | 0.040 to 4 µg |
| Ascorbic acid | Vitamin C | 0.040 to 4 µg |
| Tocopherols | Vitamin E | 0.040 to 4 µg |

## Claims

1. A composition for immunity regulation and/or stimulation for use in a method for the prevention and/or alleviation of the symptoms related to pathogenic infections comprising viral infections, bacterial infections, parasitic infections such as malaria, fungal infections, superinfections and episodic infections, per 100g or 100ml, comprising the combination of:
• 10µg to 1000µg of positively charged minerals consisting of magnesium,
• 10µg to 1000µg of metals selected from zinc and iron
• 7µg to 700µg of at least one plant oil selected from *Ribes nigrum Oleum Acini* (blackcurrant seed oil) and *Elaeis guineensis* Oleum (palm oil),
• 6µg to 600µg of at least two plant extracts selected from *Thymus vulgaris* (thyme), *Cicer arietinum* (chickpea), and *Ervum lens* (lentil),
• 6µg to 600µg of at least one alga chosen among *Fucus vesiculosus* (Fucus), *Undaria pinnatifida* (Wakame), *Palmaria palmata* (Dulse), and *Porphyra umbilicalis* (Nori),
• 7µg to 700µg of at least one mushroom chosen among *Lentinula edodes* (Shiitake) and *Grifola frondosa* (Maitake),
• 8µg to 800µg of at least one vitamin chosen among vitamins A, B1, B2, B5, B6, B9, C, E, PP(B3);
optionally with a suitable excipient.

2. The composition for use according to claim 1, wherein the input weight of vitamin A represents from 120 to 800 µg/100g of said composition.

3. The composition for use according to claim 1, wherein the weight of zinc represents from 0.225 to 1 mg/100g of said composition.

4. The composition for use according to any one of claims 1-3, wherein the suitable excipient is a pharmaceutically acceptable excipient.

5. The composition for use according to any one of claims 1 to 4, wherein said composition is a food preparation, a dietary supplement, a nutraceutical, or a beverage.

6. The composition for use according to claim 1, wherein the episodic infections comprise the winter infections; flu syndromes; bronchitis; tuberculosis; lower and upper respiratory tract infections including the Ear Nose and Throat (ENT) area; the urinary; intestinal; mucosal and skin infections.

7. The composition for use according to any one of claims 1-4, for the potentiating of antibiotics in pulmonary super infections comprising pulmonary, ENT; urinary or skin infections.

8. The composition for use according to any one of claims 1-4, for the lowering of secondary infections due to chronic pathologies.

9. The composition for use according to any one of claims 1-4, for increasing the mucosal immune defence.

10. The composition for use according to claims 8 or 9, for the treatment or prevention of chronic obstructive bronchopneumopathy disease (COPD) or Bronchiectasy.

11. The composition for use according to any one of claims 1-4, for alleviating HIV/AIDS disorders.

12. The composition for use according to any one of claims 1-4, for the regulation of inflammation due to an inappropriate immune response or to infections.

13. The composition for use according to any one of claims 1-4, for enhancing the immune response to antigenic proteins in vaccination protocols.

## Patentansprüche

1. Zusammensetzung zur Immunregulierung und/oder Stimulation zur Verwendung in einem Verfahren für die Verhütung und/oder Linderung der Symptome, die in Verbindung mit pathogenen Infektionen stehen, welche virale Infektionen, bakterielle Infektionen, parasitische Infektionen, wie zum Beispiel Malaria, Pilzinfektionen, Superinfektionen und episodische Infektionen umfassen, pro 100 g oder 100 ml, die die folgende Kombination umfasst:
• 10 µg bis 1000 µg von positiv geladenen Mineralien, die aus Magnesium bestehen,
• 10 µg bis 1000 µg von Metallen, die aus Zink und Eisen ausgewählt werden
• 7 µg bis 700 µg von mindestens einem Pflanzenöl, das ausgewählt wird aus *Ribes nigrum Oleum Acini* (Samenöl der schwarzen Johannisbeere) und *Elaeis guineensis* Oleum (Palmöl),
• 6 µg bis 600 µg von mindestens zwei Pflanzenextrakten, die ausgewählt werden aus *Thymus vulgaris* (Thymian), *Cicer arietinum* (Kichererbsen) und *Ervum lens* (Linsen),
• 6 µg bis 600 µg von mindestens einer Alge, ausgewählt aus *Fucus vesiculosus* (Fucus), *Undaria pinnatifida* (Wakame), *Palmaria palmata* (Dulse) und *Porphyra umbilicalis* (Nori),
• 7 µg bis 700 µg von mindestens einem Pilz, der ausgewählt wird aus *Lentinula edodes* (Shiitake) und *Grifola fron*dosa (Maitake),
• 8 µg bis 800 µg von mindestens einem Vitamin, das unter den Vitaminen A, B1, B2, B5, B6, B9, C, E, PP(B3) ausgewählt wird;
optional mit einem geeigneten Exzipienten.

2. Zusammensetzung zur Verwendung nach Anspruch 1, wobei das Eingangsgewicht von Vitamin A 120 bis 800 µg/100 g der Zusammensetzung repräsentiert.

3. Zusammensetzung zur Verwendung nach Anspruch 1, wobei das Gewicht von Zink 0,225 bis 1 mg/100 g der Zusammensetzung repräsentiert.

4. Zusammensetzung zur Verwendung nach einem der Ansprüche 1-3, wobei der geeignete Exzipient ein pharmazeutisch akzeptabler Exzipient ist.

5. Zusammensetzung zur Verwendung nach einem der Ansprüche 1 bis 4, wobei die Zusammensetzung eine Nahrungsmittelzubereitung, ein Nahrungsergänzungsmittel, eine medizinisch wirksame Lebensmittelmischung oder ein Getränk ist.

6. Zusammensetzung zur Verwendung nach Anspruch 1, wobei die episodischen Infektionen die Winterinfektionen; Grippe-Syndrome; Bronchitis; Tuberkulose; Infektionen der unteren und oberen Luftwege, einschließlich des Ohr-, Nasen- und Hals-Bereichs (HNO); Harnwegsinfektionen; Darmerkrankungen; Schleimhaut-und Hautinfektionen umfassen.

7. Zusammensetzung zur Verwendung nach einem der Ansprüche 1-4 zur Potenzierung von Antibiotika in pulmonalem Superinfektionen, die umfassen: Lungenerkrankungen, HNO-Erkrankungen; Harnwegsinfektionen oder Hautinfektionen.

8. Zusammensetzung zur Verwendung nach einem der Ansprüche 1-4 zur Schwächung von sekundären Infektionen aufgrund von chronischen Erkrankungen.

9. Zusammensetzung zur Verwendung nach einem der Ansprüche 1-4 zur Verstärkung der Immunabwehr der Schleimhaut.

10. Zusammensetzung zur Verwendung nach Anspruch 8 oder 9, zur Behandlung oder Verhütung von chronisch obstruktiver Bronchopneumopathie (COPD) oder Bronchiektasie.

11. Zusammensetzung zur Verwendung nach einem der Ansprüche 1-4 zur Linderung von HIV/AIDS-Erkrankungen.

12. Zusammensetzung zur Verwendung nach einem der Ansprüche 1-4 zur Regulierung der Entzündung aufgrund einer unangemessenen Immunreaktion oder von Infektionen.

13. Zusammensetzung zur Verwendung nach einem der Ansprüche 1-4 zur Verstärkung der Immunreaktion auf antigene Proteine bei Impfungen.

## Revendications

1. Composition pour la régulation et/ou la stimulation de l'immunité destinée à être utilisée dans une méthode de prévention et/ou de soulagement des symptômes liés à des infections pathogènes, comprenant les infections virales, les infections bactériennes, les infections parasitaires telles que le paludisme, les infections fongiques, les surinfections et les infections épisodiques, comprenant, pour 100 g ou 100 ml, la combinaison de :
• 10 à 1000 µg de minéraux à charge positive consistant en magnésium,
• 10 à 1000 µg de métaux choisis parmi le zinc et le fer
• 7 à 700 µg d'au moins une huile végétale choisie parmi l'huile de *Ribes nigrum Acini* (huile de pépins de cassis) et l'huile *d'Elaeis guineensis* (huile de palme),
• 6 à 600 µg d'au moins deux extraits végétaux choisis parmi *Thymus vulgaris* (thym), *Cicer arietinum* (pois chiche), et *Ervum lens* (lentille),
• 6 à 600 µg d'au moins une algue choisie parmi *Fucus vesiculosus* (Fucus), *Undaria pinnatifida* (Wakamé), *Palmaria palmata* (Dulse), et *Porphyra umbilicatis* (Nori),
• 7 à 700 µg d'au moins un champignon choisi parmi *Lentinula edodes* (Shitaké) et *Grifola frondosa* (Maitaké),
• 8 à 800 µg d'au moins une vitamine choisie parmi les vitamines A, B1, B2, B5, B6, B9, C, E, PP (B3) ;
éventuellement avec un excipient convenable.

2. Composition pour son utilisation selon la revendication 1, dans laquelle l'apport en poids de vitamine A représente de 120 à 800 µg/100 g de ladite composition.

3. Composition pour son utilisation selon la revendication 1, dans laquelle le poids de zinc représente de 0,225 à 1 mg/100 g de ladite composition.

4. Composition pour son utilisation selon l'une quelconque des revendications 1 à 3, dans laquelle l'excipient convenable est un excipient pharmaceutiquement acceptable.

5. Composition pour son utilisation selon l'une quelconque des revendications 1 à 4, dans laquelle ladite composition est une préparation alimentaire, un complément alimentaire, un neutraceutique, ou une boisson.

6. Composition pour son utilisation selon la revendication 1, dans laquelle les infections épisodiques comprennent les infections hivernales ; les syndromes grippaux ; la bronchite ; la tuberculose ; les infections des voies respiratoires inférieures et supérieures dont la zone oto-rhino-laryngologique (ORL) ; les infections urinaires ; intestinales ; des muqueuses et cutanées.

7. Composition pour son utilisation selon l'une quelconque des revendications 1 à 4, pour la potentialisation des antibiotiques dans les surinfections pulmonaires comprenant les infections pulmonaires, ORL ; urinaires ou cutanées.

8. Composition pour son utilisation selon l'une quelconque des revendications 1 à 4, pour l'abaissement des infections secondaires dues à des pathologies chroniques.

9. Composition pour son utilisation selon l'une quelconque des revendications 1 à 4, pour le renforcement de la défense immunitaire des muqueuses.

10. Composition pour son utilisation selon les revendications 8 ou 9, pour le traitement ou la prévention de la bronchopneumopathie chronique obstructive (BPCO) ou de la bronchiectasie.

11. Composition pour son utilisation selon l'une quelconque des revendications 1 à 4, pour le soulagement des troubles liés au VIH/SIDA.

12. Composition pour son utilisation selon l'une quelconque des revendications 1 à 4, pour la régulation de l'inflammation due à une réponse immunitaire inappropriée ou à des infections.

13. Composition pour son utilisation selon l'une quelconque des revendications 1 à 4, pour le renforcement de la réponse immunitaire à des protéines antigéniques dans des protocoles de vaccination.
